# EUROPEAN PATENT APPLICATION

(11) **EP 1 158 049 A1**
(43) Date of publication of application: **28.11.2001**
(21) Application number: 01112637.2
(22) Date of filing: 24.05.2001
(51) Int. Cl.: C12N 15/12, C12N 15/62, C07K 14/47, C07K 16/18, C12Q 1/68, G01N 33/53, A61K 38/17, A61K 31/7088

(54) **Myosin-like gene expressed in human heart and muscle**

(30) Priority: 26.05.2000 US 207456 P; 05.02.2001 US 266860 P
(71) Applicant: Aeomica, INC., Sunnyvale, CA 94086 (US)
(72) Inventor: Gu, Yizhong, Sunnyvale, CA 94087 (US); Ji, Yonggang, San Mateo, CA 94403 (US); Penn, Sharron G., San Mateo, CA 94402 (US); Hanzel, David K., Palo Alto, CA 94303 (US)
(74) Representative: Canning, Lewis R.

(57) **Abstract**

The invention provides compounds and compositions comprising isolated nucleic acids encoding a novel human myosin-like protein particularly expressed in heart and muscle, compositions derivable directly or indirectly therefrom, including probes, proteins, and antibodies, and methods for using the same.

## Description

### FIELD OF THE INVENTION

The present invention relates to isolated nucleic acids encoding a novel human myosin-like gene particularly expressed in heart and muscle, compounds and compositions derivable directly or indirectly therefrom, and methods for using the same.

### BACKGROUND OF THE INVENTION

Myosins are proteins that act as ubiquitous intracellular engines, typically motoring along tracks of actin filaments to drive a variety of cellular processes including muscular contraction, cytokinesis, membrane trafficking and signal transduction. Baker *et al*., *Curr*. *Opin*. *Cell Biol.* 10: 80-86 (1998). Given the range of intracellular passengers and itineraries, some form of the myosin protein is found in virtually all eukaryotic cells.

The myosin gene superfamily has at least seventeen members, or classes, encoded by multiple genes.

Mammalian cells have the largest number of myosin genes, with the 28 identified myosin genes belonging to nine classes. Sellers, *Biochim*. *Biophys*. *Acta 1496:* 3-22 (2000). The genome of the yeast, *Saccharomyces cerevisiae,* contains just 5 myosin genes. Brown, *Curr*. *Opin*. *Cell Biol*. 9: 44-48 (1997). Between these extremes, the nematode *Caenorhabditis elegans* has 14 identified myosin genes. Baker *et al*., *J*. *Mol. Biol.* 172: 523-535 (1997). Myosins are also found in plant cells, with myosin genes in classes VIII, XI and XIII expressed exclusively in plants.

The structure of myosin always includes one or two heavy chains and several light chains.

The heavy chain is composed of three structurally and functionally different domains. The head domain, which is highly conserved across the myosin family, functions to generate force, and contains actin-binding and ATP-binding sites; the ATPase activity of the head domain is activated by actin binding. The neck region, which mediates association with the light chains, regulates the adjacent head domain. The tail domain regulates the specific activity of each myosin.

The light chains of myosin I and myosin V are calmodulin, which is a calcium-binding regulatory subunit in certain enzymes. Myosin II is also regulated by calcium-binding light chains, but not calmodulin.

Together, the disparate myosin heavy and light chains permit myosins to serve disparate cellular roles.

For example, in brush border microvilli, myosin I, one of the two most abundant forms of myosin, links the microfilament bundles to the plasma membrane. Myosin II, the other of the two most abundant myosin classes, forms dimers in muscle cells that associate to form thick filaments, which are part of the contractile apparatus.

In addition to its well characterized role in contraction and force production in skeletal, cardiac, and smooth muscles, myosin II is required for cytokinesis, cell motility, cell polarity/chemotaxis, maintaining cell architecture and development in nonmuscle cells. Sellers, *Biochim*. *Biophys*. *Acta* 1496:3-22 (2000).

Thus, contractile bundles, which comprise both actin and myosin II filaments, are found in numerous cell types. In epithelial cells, these bundles are called the circumferential belt and can function structurally (*e.g.*, as an internal brace helping to control cell shape), or for motility (*e.g*., in wound healing, contraction seals the gap in a sheet of cells).

Myosin II is an integral component to the cytoskeletal substructure, acting to stiffen cortical membranes. In cytokinesis, myosin II plays an essential role performing the motor function for the contractile ring which constricts to form the cleavage furrow.

Myosin IXs, identified in rat, human and *C. elegans,* are expressed in a wide variety of tissues and cell types and are believed to be involved in intracellular signaling pathways. Myosin IX acts as a negative regulator of Rho (a G-protein), suggesting that it may control the Rho signaling pathways involved in cytoskeleton reorganization and other cellular processes. Bahler *et al*., *Biochim*. *Biophys*. *Acta* 1496:52-59 (2000). However the precise cellular functions of the myosin IXs and their exact roles in the Rho signaling pathways have still to be determined.

Membrane-bound myosins of various classes, particularly myosin I and myosin V, have been implicated in movement of vesicles within the cell. Due to its co-localization with Golgi membrane, myosin I is thought to move membrane vesicles between membrane compartments in the cytoplasm. Myosin I also serves as a membrane-microfilament linkage in microvilli.

In yet other examples of myosin function, protein secretion in yeast is disrupted by mutation in the myosin V gene, suggesting an important role for myosin V. In vertebrate brain tissue, myosin V is concentrated in the Golgi stacks and at the tips of membrane processes extending from neuronal cells. Espreafico *et al*., *J*. *Cell Biol.* 119: 1541 (1992). This type of membrane association would be consistent with the effects of myosin V mutations in mice, which adversely affect synaptic transmission, resulting in seizures and eventually death. In cell migration, different myosins localize to different regions of the cell. Myosin I localizes to the leading edge of a crawling amoeba, possibly participating in the translocation phase, whereas myosin II is more concentrated at the tail, where it is involved in retraction of the cell body.

Given their ubiquity, and the wide variety of tasks driven by myosins, it is not surprising that myosin defects have been implicated in a wide variety of diseases.

For example, as noted above, myosin V mutations in mice adversely affect synaptic transmission, resulting in seizures and eventually death.

Myosin and myosin-like genes have also been implicated in a number of human diseases.

For example, myosin plays a role in hypertrophic cardiomyopathy. An autosomally dominant form of the disease is frequently caused by a missense point mutation in exon 13 of the cardiac myosin heavy chain gene on chromosome 14. Less often, an abnormal cardiac myosin heavy chain hybrid gene is present.

As another example, mutation in the gene encoding myosin VIIA is responsible for some forms of Usher syndrome. Weil *et al., Nature* 374:60-61 (1995). Usher Syndrome is characterized by hearing impairment associated with retinitis pigmentosa.

Three classes of myosins, VI, VII and XV have been associated with genetic deafness disorders in mammals. Hasson, *Am. J. Hum. Genet.* 61:801-5 (1997); Redowicz, *J. Muscle Res. Cell Mot.* 20:241-248 (1999). Mutations in these myosins result in abnormalities in the stereocilia in the sensory cells of the inner ear of mice.

Other myosin-like genes may also be needed for normal stereociliary function in the inner ear, and mutations in these additional myosin-like genes may thus underlie or contribute to various forms of congenital deafness. For example, nonsyndromic hereditary deafness (DFNA17), mapped to chromosome 22q12.2-q13.3 in 1997, has now been associated with mutation in MYH9, a nonmuscle-myosin heavy-chain gene, located within the linked region. Lalwani *et al., Am. J. Hum. Genet.* 67:1121-8 (2000).

Despite the long-standing interest in myosins and sequence similarity among classes and across species, not all myosin and myosin-like genes have yet been identified, even in species that are genetically well characterized.

For example, a novel myosin-like gene (MysPDZ) has recently been cloned from mouse bone marrow stromal cells. The protein encoded by this newly identified gene contains a PDZ domain but no actin-binding domain. Furusawa *et al*., *Biochem. Biophys*. *Res*. *Comm.* 270: 67-75 (2000). PDZ domains are implicated in modular protein-protein interactions, also binding to specific C-terminal sequences of membrane proteins. Gee *et al*., *Biochem.* 39: 14638-46 (2000).

MysPDZ appears to localize in the cytoskeleton. Furusawa *et al.* show significant similarity between MysPDZ and a genomic clone from human chromosome 17; a human orthologue has been identified which contains an ATP/GTP-binding site. Nagase *et al*., *DNA Res.* 3: 321-329 (1996). Another PDZ-domain containing protein in humans has been shown to associate with actin filaments, possibly through binding to alpha-actinin. Bauer *et al*., *Blood* 96: 4236-45 (2000).

Given the importance of myosins in normal cellular processes and in disease, there is a need in the art for access to as yet undiscovered myosin and myosin-like genes.

### SUMMARY OF THE INVENTION

These and other needs in the art are satisfied by the present invention, which provides compounds, compositions and methods based upon the identification and cloning of a novel human myosin-like protein, hGDMLP-1, which is expressed at high levels in human heart and skeletal muscle.

In a first aspect, the invention provides an isolated nucleic acid molecule selected from the group consisting of: (a) an isolated nucleic acid molecule that encodes the amino acid sequence of human genome-derived myosin-like protein, SEQ ID NO:2; (b) an isolated nucleic acid molecule that encodes a fragment of at least 10 amino acids of SEQ ID NO:2; (c) an isolated nucleic acid molecule that encodes an amino acid sequence having at least about 65% amino acid sequence identity to SEQ ID NO:2; (d) an isolated nucleic acid which hybridizes under high stringency conditions to a nucleic acid molecule comprising SEQ ID NO:1; and (e) an isolated nucleic acid complementary in sequence to the nucleic acids of (a) - (d). In one embodiment, the isolated nucleic acid comprises sequence of SEQ ID NO:1 or its complement.

In various related aspects, the invention provides a vector comprising the nucleic acids of the present invention. In certain embodiments, the vector is an expression vector. The invention further provides a host cell transformed to contain the nucleic acid molecules of the present invention, and methods for producing a polypeptide, the method comprising: culturing the transformed host cell of the present invention under conditions in which the protein encoded by the transformed nucleic acid molecule is expressed, the method optionally including a later step of isolating the expressed polypeptide.

In another aspect, the invention provides an isolated polypeptide produced by the above-described method. The invention further provides an isolated polypeptide selected from the group consisting of: (a) an isolated polypeptide comprising the amino acid sequence of SEQ ID NO:2; (b) an isolated polypeptide comprising a fragment of at least 10 amino acids of SEQ ID NO:2; (c) an isolated polypeptide according to (a) or (b) in which at least 95% of deviations from the sequence of (a) or (b) are conservative substitutions; and (d) an isolated polypeptide having at least 65% amino acid sequence identity to the isolated polypeptides of (a) or (b).

In a yet further aspect, the invention provides an isolated antibody or antigen-binding fragment or derivative thereof, the binding of which can be competitively inhibited by a polypeptide of the present invention.

In other aspects, the invention provides the aforesaid nucleic acid molecules attached to a support, and in a related aspect, further provides a microarray wherein at least one probe of the array is a nucleic acid according to the invention.

In an additional aspect, the invention provides a method of identifying binding partners for a polypeptide of the present invention, the method comprising: contacting the polypeptide to a potential binding partner; and determining if the potential binding partner binds to the polypeptide. In some embodiments the contacting is performed *in vivo.*

In yet another aspect, the invention provides a method of modulating the expression of a nucleic acid of the present invention, the method comprising: administering an effective amount of an agent which modulates the expression of a nucleic acid of the present invention.

In another aspect, the invention provides a method of modulating at least one activity of a polypeptide of the present invention, the method comprising: administering an effective amount of an agent which modulates at least one activity of a polypeptide of the present invention.

The invention further provides a transgenic non-human animal modified to contain a nucleic acid molecule of the present invention. In some embodiments, the transgenic non-human animal is unable to express the polypeptide of the present invention.

The invention also provides, in a further aspect, a method of diagnosing a disease caused by mutation in hGDMLP-1, comprising: detecting the mutation in a sample of nucleic acids that derives from a subject suspected to have the disease.

The invention provides a method of diagnosing or monitoring a disease caused by altered expression of hGDMLP-1, comprising: determining the level of expression of hGDMLP-1 in a sample of nucleic acids or proteins that derives from a subject suspected to have the disease, alterations from a normal level of expression providing diagnostic and/or monitoring information.

In another aspect, the invention provides pharmaceutical compositions.

In a first such aspect, the invention provides a pharmaceutical composition comprising the nucleic acid of the present invention. In a second such aspect, the invention provides a pharmaceutical composition comprising the polypeptide of the present invention. In yet another such aspect, the invention provides a pharmaceutical composition comprising the antibody or antigen-binding fragment or derivative thereof of the present invention.

The invention further provides purified agonists and antagonists of the polypeptide of the present invention, and pharmaceutical compositions thereof.

The invention provides a method for treating or preventing a disorder associated with decreased expression or activity of hGDMLP-1, the method comprising administering to a subject in need of such treatment an effective amount of the pharmaceutical composition comprising any one of the nucleic acid, polypeptide, or agonist of the present invention. Conversely, the invention provides a method for treating or preventing a disorder associated with increased expression or activity of hGDMLP-1, the method comprising administering to a subject in need of such treatment an effective amount of the pharmaceutical composition comprising the antibody or antagonist of the present invention.

The invention further comprises diagnostic compositions, the diagnostic compositions comprising any of the nucleic acid, polypeptide, and antibody of the present invention. In certain embodiments, the diagnostic composition is suitable for in vivo administration.

In yet another aspect, the invention provides a method for detecting a target nucleic acid in a sample, the target being a nucleic acid of the present invention, the method comprising: a) hybridizing the sample with a probe comprising at least 30 contiguous nucleotides of a sequence complementary to the target nucleic acid in the sample under hybridization conditions sufficient to permit detectable binding of the probe to the target, and b) detecting the presence or absence, and optionally the amount, of such binding.

In a further aspect, the invention provides a fusion protein, the fusion protein comprising a polypeptide of the present invention fused to a heterologous amino acid sequence.
In certain embodiments, the heterologous amino acid sequence is a detectable moiety. In a preferred embodiment, the detectable moiety is fluorescent. In other embodiments, the heterologous amino acid sequence is an Ig Fc region.

The invention provides a method of screening for agents that modulate the expression of hGDMLP-1, the method comprising: contacting a cell or tissue sample believed to express hGDMLP-1 with a chemical or biological agent, and then comparing the amount of hGDMLP-1 expression with that of a control.

### BRIEF DESCRIPTION OF THE DRAWINGS

The above and other objects and advantages of the present invention will be apparent upon consideration of the following detailed description taken in conjunction with the accompanying drawings, in which like characters refer to like parts throughout, and in which:
FIG. 1 is a screen shot of a graphical display that facilitates identification of exons in genomic sequence the expression of which meets user criteria, particularly identifying heart-specific expression of one exon of the gene of the present invention;
FIGS. 2A - 2L collectively show the sequence of the cDNA encoding the human genome-derived myosin-like protein (hGDMLP-1) of the present invention, with its predicted amino acid sequence;
FIGS. 3A - 3C collectively show an alignment of the hGDMLP-1 coding sequence of the present invention with the two closest known orthologues;
FIG. 4 shows an alignment of the amino acid sequence of hGDMLP-1 of the present invention and human myocilin;
FIG. 5 schematizes the genomic organization of the hGDMLP-1 gene, showing the relative location and size of the 44 exons of the protein and their relationship to the genomic clones (BACs) and cDNA open reading frame; and
FIG. 6 is a northern blot probed with a labeled fragment of hGDMLP-1, according to the present invention, showing muscle- and heart-specific expression of an 8 kb transcript corresponding to hGDMLP-1.

### DETAILED DESCRIPTION OF THE INVENTION

Before the proteins, nucleotide sequences, and methods of the present invention are described, it is to be understood that this invention is not limited to the particular methodology, protocols, cell lines, vectors, and reagents described, as these can be varied. It is also to be understood that the terminology used herein is for the purpose of describing particular embodiments only, and is not intended to limit the scope of the present invention which will be limited only by the appended claims.

As used herein and in the appended claims, the singular forms "a", "an", and "the" include plural reference unless the context clearly dictates otherwise. Thus, for example, reference to "a host cell" includes a plurality of such host cells, reference to the "antibody" is a reference to one or more antibodies and equivalents thereof known to those skilled in the art, and so forth.

Unless defined otherwise, all technical and scientific terms used herein have the same meanings as commonly understood by one of ordinary skill in the art to which this invention belongs.

### Definitions

As used herein, the term "microarray" and equivalent phrase "nucleic acid microarray" refer to a substrate-bound collection of plural nucleic acids, hybridization to each of the plurality of bound nucleic acids being separately detectable. The substrate can be solid or porous, planar or non-planar, unitary or distributed.

As so defined, the term "microarray" and phrase "nucleic acid microarray" include all the devices so called in Schena (ed.), DNA Microarrays: A Practical Approach (Practical Approach Series), Oxford University Press (1999) (ISBN: 0199637768); *Nature Genet.* 21(1)(suppl):1 - 60 (1999); and Schena (ed.), Microarray Biochip: Tools and Technology, Eaton Publishing Company/BioTechniques Books Division (2000) (ISBN: 1881299376), the disclosures of which are incorporated herein by reference in their entireties.

As so defined, the term "microarray" and phrase "nucleic acid microarray" also include substrate-bound collections of plural nucleic acids in which the nucleic acids are distributably disposed on a plurality of beads, rather than on a unitary planar substrate, as is described, *inter alia,* in Brenner *et al*., *Proc*. *Natl*. *Acad*. *Sci*. *USA* 97(4):166501670 (2000), the disclosure of which is incorporated herein by reference in its entirety; in such case, the term "microarray" and phrase "nucleic acid microarray" refer to the plurality of beads in aggregate.

As used herein with respect to a nucleic acid microarray, the term "probe" refers to the nucleic acid that is, or is intended to be, bound to the substrate. As used herein with respect to solution phase hybridization, the term "probe" refers to the nucleic acid of known sequence that is, or is intended to be, detectably labeled. In either such context, the term "target" refers to nucleic acid intended to be bound to probe by Watson-Crick complementarity. As used herein, the expression "probe comprising SEQ ID NO", and variants thereof, intends a nucleic acid probe, at least a portion of which probe has either (i) the sequence directly as given in the referenced SEQ ID NO, or (ii) a sequence complementary to the sequence as given in the referenced SEQ ID NO, the choice as between sequence directly as given and complement thereof dictated by the requirement that the probe be complementary to the desired target.

As used herein, the phrase "expression of a probe" and its linguistic variants means that the probe hybridizes detectably at high stringency to nucleic acids that derive from mRNA.

As used herein, the term "exon" refers to a nucleic acid sequence bioinformatically predicted to encode a portion of a natural protein.

As used herein, the phrase "open reading frame" and the equivalent acronym "ORF" refer to that portion of an exon that can be translated in its entirety into a sequence of contiguous amino acids. As so defined, an ORF is wholly contained within its respective exon and has length, measured in nucleotides, exactly divisible by 3. As so defined, an ORF need not encode the entirety of a natural protein.

As used herein, the phrase "alternative splicing" and its linguistic equivalents includes all types of RNA processing that lead to expression of plural protein isoforms from a single gene; accordingly, the phrase "splice variant(s)" and its linguistic equivalents embraces mRNAs transcribed from a given gene that, however processed, collectively encode plural protein isoforms. For example, and by way of illustration only, splice variants can include exon insertions, exon extensions, exon truncations, exon deletions, alternatives in the 5' untranslated region ("5' UT") and alternatives in the 3' untranslated region ("3' UT"). Such 3' alternatives include, for example, differences in the site of RNA transcript cleavage and site of poly(A) addition. See, *e.g.,* Gautheret *et al., Genome Res.* 8:524-530 (1998).

As used herein, "orthologues" are separate occurrences of the same gene in multiple species. The separate occurrences have similar, albeit nonidentical, amino acid sequences, the degree of sequence similarity depending, in part, upon the evolutionary distance of the species from a common ancestor having the same gene.

As used herein, the term "paralogues" indicates separate occurrences of a gene in one species. The separate occurrences have similar, albeit nonidentical, amino acid sequences, the degree of sequence similarity depending, in part, upon the evolutionary distance from the gene duplication event giving rise to the separate occurrences.

As used herein, the term "homologues" is generic to "orthologues" and "paralogues".

Unless otherwise clear from the context, "hGDMLP-1", as used herein, refers both to the amino acid sequences of substantially purified hGDMLP-1 obtained from human beings, and additionally to orthologues thereof from any species, particularly mammalian species, including bovine, ovine, porcine, murine, rat, and equine species, from any source whether natural, synthetic, semi- synthetic, or recombinant.

The term "agonist", as used herein, refers to a molecule which, when bound to hGDMLP-1, increases or prolongs the duration of the effect of hGDMLP-1, or stimulates the function of hGDMLP-1. Agonists can include proteins, nucleic acids, carbohydrates, or any other molecules which bind to and modulate the effect of hGDMLP-1.

An "allele" or "allelic sequence", as used herein, is an alternative form of the gene encoding hGDMLP-1. Alleles may result from at least one mutation in the nucleic acid sequence and may result in altered mRNAs or polypeptides whose structure or function may or may not be altered. Any given natural or recombinant gene may have none, one, or many allelic forms. Common mutational changes which give rise to alleles are generally ascribed to natural deletions, additions, or substitutions of nucleotides. Each of these types of changes may occur alone, or in combination with the others, one or more times in a given sequence. "Altered" nucleic acid sequences encoding hGDMLP-1, as used herein include those with deletions, insertions, or substitutions of different nucleotides resulting in a polynucleotide that encodes the same or a functionally equivalent hGDMLP-1. Included within this definition are polymorphisms which may or may not be readily detectable using a particular oligonucleotide probe of the polynucleotide encoding hGDMLP-1, and improper or unexpected hybridization to alleles, with a locus other than the normal chromosomal locus for the polynucleotide sequence encoding hGDMLP-1. The encoded protein may also be "altered" and contain deletions, insertions, or substitutions of amino acid residues which produce a silent change and result in a functionally equivalent hGDMLP-1. Deliberate amino acid substitutions may be made on the basis of similarity in polarity, charge, solubility, hydrophobicity, hydrophilicity, and/or the amphipathic nature of the residues as long as the biological or immunological activity of hGDMLP-1 is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine, glycine and alanine, asparagine and glutamine, serine and threonine, and phenylalanine and tyrosine.

"Amino acid sequence", as used herein refers to an oligopeptide, peptide, polypeptide, or protein sequence, and fragment thereof, and to naturally occurring or synthetic molecules. Fragments of hGDMLP-1 are preferably about 10 to about 15 amino acids in length and retain the biological activity or the immunological activity of hGDMLP-1. Where "amino acid sequence" is recited herein to refer to an amino acid sequence of a naturally occurring protein molecule, amino acid sequence, and like terms, are not meant to limit the amino acid sequence to the complete, native amino acid sequence associated with the recited protein molecule.

"Amplification", as used herein refers to the production of additional copies of a nucleic acid sequence and can be carried out techniques well known in the art, including polymerase chain reaction (PCR) (Dieffenbach, C.W. and G. S. Dveksler (1995) PCR Primer, a Laboratory Manual, Cold Spring Harbor Laboratory Press, Plainview, N.Y.) and rolling circle amplification (U.S. Patent Nos. 5,854,033 and 5,714,320 and international patent publications WO 97/19193 and WO 00/15779).

The term "antagonist", as used herein, refers to a molecule which, when bound to hGDMLP-1, decreases the amount or the duration of the effect of the biological or immunological activity of hGDMLP-1, or reduces the function of hGDMLP-1. Antagonists may include proteins, nucleic acids, carbohydrates, antibodies or any other molecules which decrease the effect of hGDMLP-1.

As used herein, the term "antibody" refers to intact molecules as well as fragments thereof, such as Fab and F(ab')₂, and derivatives thereof, such as scFV, which are capable of binding the epitopic determinant. Antibodies that bind hGDMLP-1 polypeptides can be prepared using intact polypeptides or fragments containing small peptides of interest as the immunizing antigen. The polypeptide or oligopeptide used to immunize an animal can be derived from the translation of RNA or synthesized chemically and can be conjugated to a carrier protein, if desired. Commonly used carriers that are chemically coupled to peptides include bovine serum albumin and thyroglobulin, keyhole limpet hemocyanin. The coupled peptide is then used to immunize the animal (e.g., a mouse, a rat, or a rabbit).

The term "antigenic determinant", as used herein, refers to that fragment of a molecule (i.e., an epitope) that makes contact with a particular antibody. When a protein or fragment of a protein is used to immunize a host animal, numerous regions of the protein may induce the production of antibodies which bind specifically to a given region or three-dimensional structure on the protein; these regions or structures are referred to as antigenic determinants. An antigenic determinant may compete with the intact antigen (i.e., the immunogen used to elicit the immune response) for binding to an antibody.

The term "antisense", as used herein, refers to any composition containing nucleotide sequences which are complementary to a specific DNA or RNA sequence. The term "antisense strand" is used in reference to a nucleic acid strand that is complementary to the "sense" strand. Antisense molecules include peptide nucleic acids and may be produced by any method including synthesis or transcription. Once introduced into a cell, the complementary nucleotides combine with natural sequences produced by the cell to form duplexes and block either transcription or translation. The designation "negative" is sometimes used in reference to the antisense strand, and "positive" is sometimes used in reference to the sense strand.

The term "biologically active", as used herein, refers to a protein having structural, regulatory, or biochemical functions of a naturally occurring molecule. Likewise, "immunologically active" refers to the capability of the natural, recombinant, or synthetic hGDMLP-1, or any oligopeptide thereof, to induce a specific immune response in appropriate animals or cells and to bind with specific antibodies.

The terms "complementary" or "complementarity", as used herein, refer to the natural duplex binding of polynucleotides under permissive salt and temperature conditions by Watson-Crick base-pairing. The degree of complementarity between nucleic acid strands has significant effects on the efficiency and strength of hybridization between nucleic acid strands. This is of particular importance in amplification reactions, which depend upon binding between nucleic acids strands and in the design and use of PNA molecules.

A "composition comprising a given polynucleotide sequence", as used herein refers broadly to any composition containing the given polynucleotide sequence. The composition may comprise a dry formulation or an aqueous solution. Compositions comprising polynucleotide sequences encoding hGDMLP-1 or fragments thereof may be employed as hybridization probes. The probes may be stored in freeze-dried form and may be associated with a stabilizing agent such as a carbohydrate. In hybridizations, the probe may be deployed in an aqueous solution containing salts (e.g., NaCl), detergents (e.g., SDS) and other components (e.g., Denhardt's solution, dry milk, salmon sperm DNA, etc.).

"Consensus", as used herein, refers to a nucleic acid sequence which has been resequenced to resolve uncalled bases, has been extended *(e.g.* by RACE or anchored PCR), or has been assembled from the overlapping sequences of more than one clone using a computer program for fragment assembly. Sequences can have been extended and assembled to produce the consensus sequence.

The term "correlates with expression of a polynucleotide", as used herein, indicates that the detection of the presence of ribonucleic acid that is similar to SEQ ID NO:1 by northern analysis is indicative of the presence of mRNA encoding hGDMLP-1 in a sample and thereby correlates with expression of the transcript from the polynucleotide encoding the protein.

A "deletion", as used herein, refers to a change in the amino acid or nucleotide sequence and results in the absence of one or more amino acid residues or nucleotides.

The term "derivative", as used herein, refers to the chemical modification of a nucleic acid encoding or complementary to hGDMLP-1 or the encoded hGDMLP-1. Such modifications include, for example, replacement of hydrogen by an alkyl, acyl, or amino group. A nucleic acid derivative encodes a polypeptide which retains the biological or immunological function of the natural molecule. A derivative polypeptide is one which is modified by glycosylation, pegylation, or any similar process which retains the biological or immunological function of the polypeptide from which it was derived.

The term "homology", as used herein, refers to a degree of sequence similarity.

Human artificial chromosomes (HACs) are linear microchromosomes which may contain DNA sequences of 10K to 10M basepairs in size and contain all of the elements required for stable mitotic chromosome segregation and maintenance (Harrington, J. J. et al. (1997) Nat Genet. 15:345-355).

The term "humanized antibody", as used herein, refers to nonhuman antibody molecules in which amino acids have been replaced in the non-antigen binding regions in order to more closely resemble a human antibody, while still retaining the original binding ability.

The term "hybridization", as used herein, refers to any process by which a strand of nucleic acid binds with a complementary strand through base pairing.

The term "hybridization complex", as used herein, refers to a complex formed between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (e.g., C₀t or R₀t analysis) or between one nucleic acid sequence present in solution and another nucleic acid sequence immobilized on a solid support (e.g., paper, membranes, filters, chips, pins or glass slides, or any other appropriate substrate to which cells or their nucleic acids have been fixed).

An "insertion" or "addition", as used herein, refers to a change in an amino acid or nucleotide sequence resulting in the addition of one or more amino acid residues or nucleotides, respectively, as compared to the naturally occurring molecule.

The term "modulate", as used herein, refers to a change in the activity of hGDMLP-1. For example, modulation may cause an increase or a decrease in protein activity, binding characteristics, or any other biological, functional or immunological properties of hGDMLP-1.

"Nucleic acid sequence", as used herein refers to an oligonucleotide, nucleotide, or polynucleotide, and fragments thereof, and to DNA or RNA of genomic or synthetic origin which may be single- or double-stranded, and represent the sense or antisense strand. "Fragments" of hGDMLP-1 are typically at least 30 nucleotides in length, at least 100 nucleotides or at least 1000 nucleotides in length, and at least 10,000 nucleotides in length.

The term "oligonucleotide" refers to a nucleic acid sequence of at least about 6 nucleotides to about 60 nucleotides, preferably about 15 to 30 nucleotides, and more preferably about 20 to 25 nucleotides, which can be used in PCR amplification or a hybridization assay, or a microarray. As used herein, oligonucleotide is substantially equivalent to the terms "amplimers", "primers", "oligomers", and "probes", as commonly defined in the art.

"Peptide nucleic acid", PNA as used herein, refers to an antisense molecule or anti-gene agent which comprises an oligonucleotide of at least five nucleotides in length linked to a peptide backbone of amino acid residues which ends in lysine. The terminal lysine confers solubility to the composition. PNAs may be pegylated to extend their life span in the cell where they preferentially bind complementary single stranded DNA and RNA and stop transcript elongation (Nielsen, P. E. et al. (1993) Anticancer Drug Des. 8:53-63).

The term "portion", as used herein, with regard to a protein (as in "a portion of a given protein") refers to fragments of that protein. The fragments may range in size from five amino acid residues to the entire amino acid sequence minus one amino acid. Thus, a protein "comprising at least a portion of the amino acid sequence of SEQ ID NO:2" encompasses the full-length hGDMLP-1 and fragments thereof.

The term "sample", as used herein, is used in its broadest sense. A biological sample suspected of containing nucleic acid encoding hGDMLP-1, or fragments thereof, or hGDMLP-1 itself may comprise a bodily fluid, extract from a cell, chromosome, organelle, or membrane isolated from a cell, a cell, genomic DNA, RNA, or cDNA (in solution or bound to a solid support, a tissue, a tissue print, and the like).

The terms "specific binding" or "specifically binding", as used herein, refers to that interaction between a protein or peptide and an agonist, an antibody and an antagonist. The interaction is dependent upon the presence of a particular structure (i.e., the antigenic determinant or epitope) of the protein recognized by the binding molecule. For example, if an antibody is specific for epitope "A", the presence of a protein containing epitope A (or free, unlabeled A) in a reaction containing labeled "A" and the antibody will reduce the amount of labeled A bound to the antibody.

The terms "stringent conditions" or "stringency", as used herein, refer to the conditions for hybridization as defined by the nucleic acid, salt, and temperature. These conditions are well known in the art and may be altered in order to identify or detect identical or related polynucleotide sequences. Numerous equivalent conditions comprising either low or high stringency depend on factors such as the length and nature of the sequence (DNA, RNA, base composition), nature of the target (DNA, RNA, base composition), milieu (in solution or immobilized on a solid substrate), concentration of salts and other components (e.g., formamide, dextran sulfate and/or polyethylene glycol), and temperature of the reactions (within a range from about 5°C below the melting temperature of the probe to about 20°C to 25°C below the melting temperature). One or more factors may be varied to generate conditions of either low or high stringency different from, but equivalent to, the above listed conditions.

The term "substantially purified", as used herein, refers to nucleic or amino acid sequences that are removed from their natural environment, isolated or separated, and are at least 60% free, preferably at least 75% free, and most preferably at least 90% free from other components with which they are naturally associated.

A "substitution", as used herein, refers to the replacement of one or more amino acids or nucleotides by different amino acids or nucleotides, respectively.

"Transformation", as defined herein, describes a process by which exogenous DNA enters and changes a recipient cell. It may occur under natural or artificial conditions using various methods well known in the art. Transformation may rely on any known method for the insertion of foreign nucleic acid sequences into a prokaryotic or eukaryotic host cell. The method is selected based on the type of host cell being transformed and may include, but is not limited to viral infection, electroporation, heat shock, lipofection, and particle bombardment. Such "transformed" cells include stably transformed cells in which the inserted DNA is capable of replication either as an autonomously replicating plasmid or as part of the host chromosome. They also include cells which transiently express the inserted DNA or RNA for limited periods of time.

A "variant" of hGDMLP-1, as used herein, refers to an amino acid sequence that is altered by one or more amino acids. The variant may have "conservative" changes, wherein a substituted amino acid has similar structural or chemical properties, e.g., replacement of leucine with isoleucine. Other variants may have "nonconservative" changes, e.g., replacement of a glycine with a tryptophan. Analogous minor variations may also include amino acid deletions or insertions, or both. Guidance in determining which amino acid residues may be substituted, inserted, or deleted without abolishing biological or immunological activity may be found using computer programs well known in the art, for example, LASERGENE software.
hGDMLP-1

The invention is based on the discovery of a new human myosin heavy chain-like protein, hGDMLP-1, the polynucleotides encoding hGDMLP-1, and the use of these compositions for the diagnosis, prevention, or treatment of disorders.

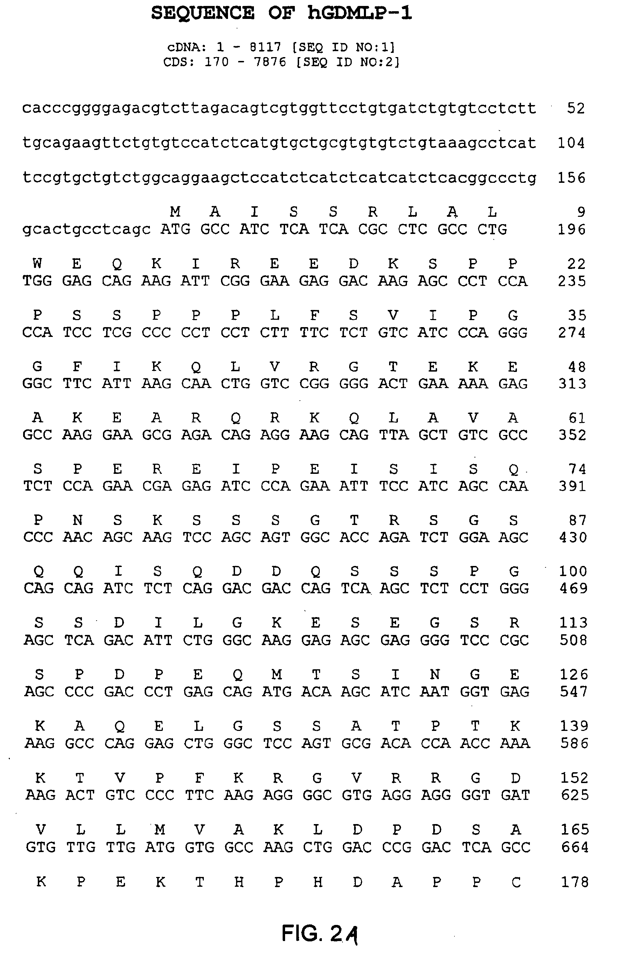

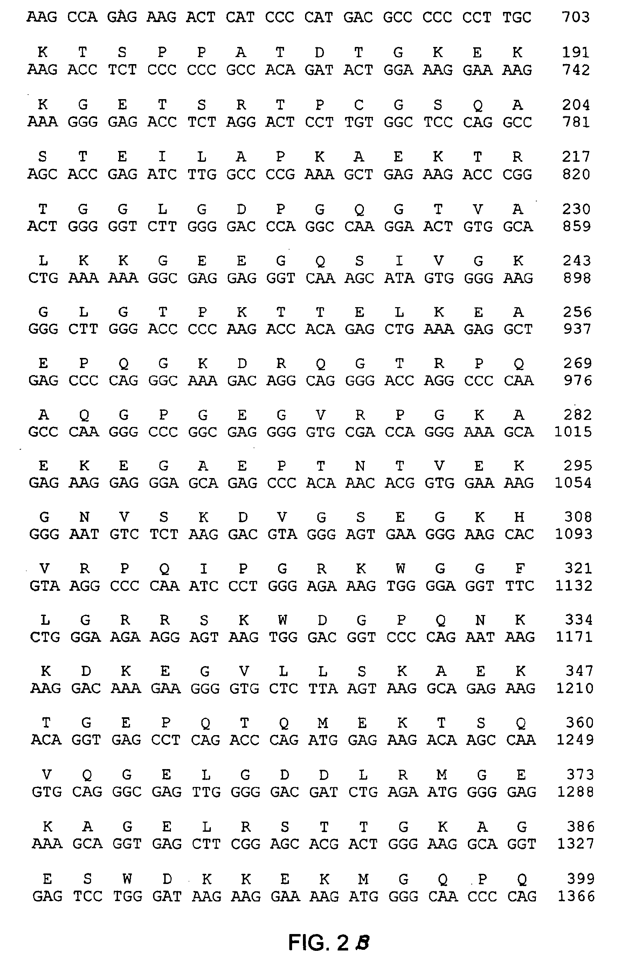

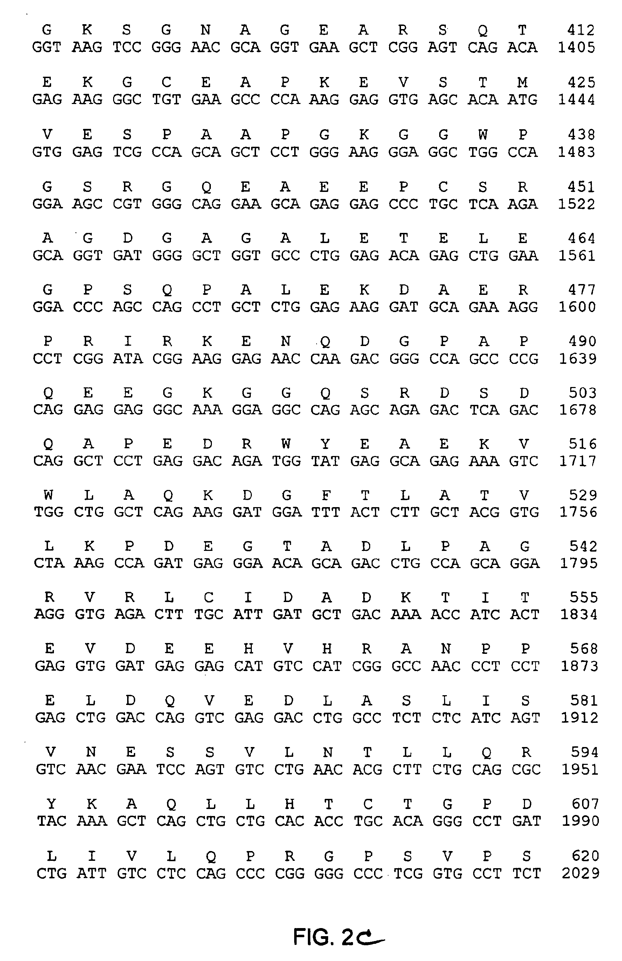

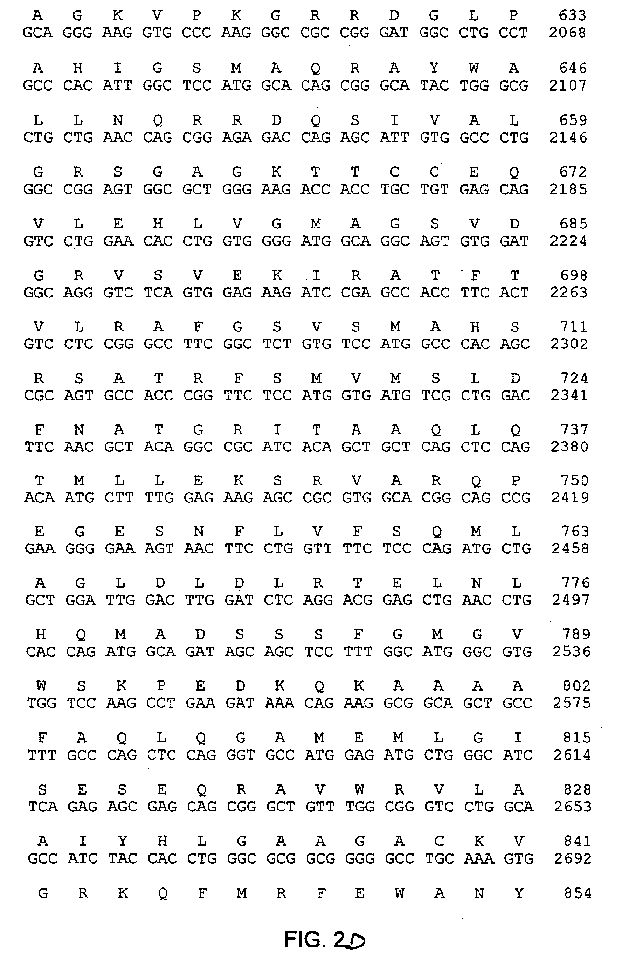

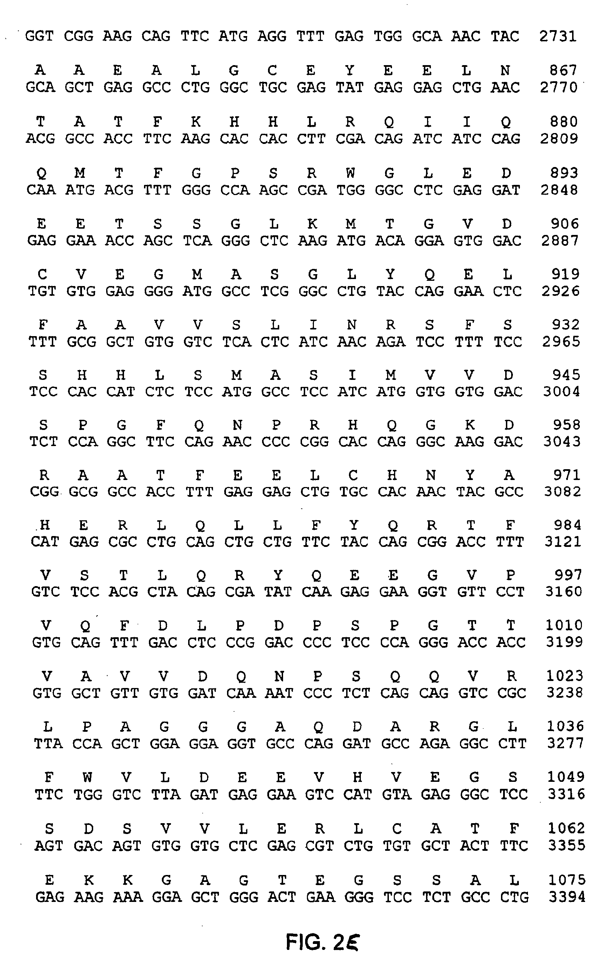

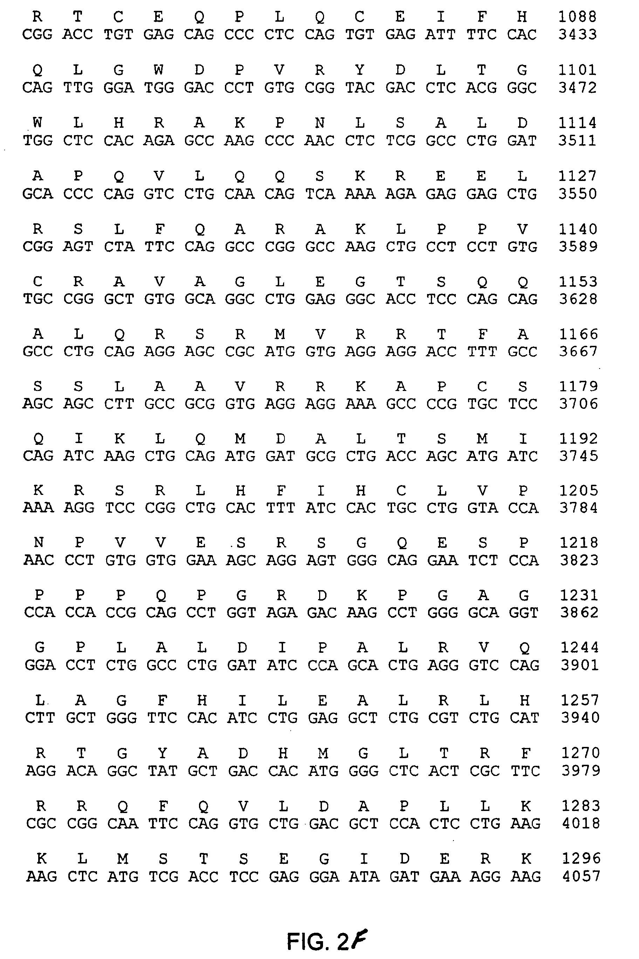

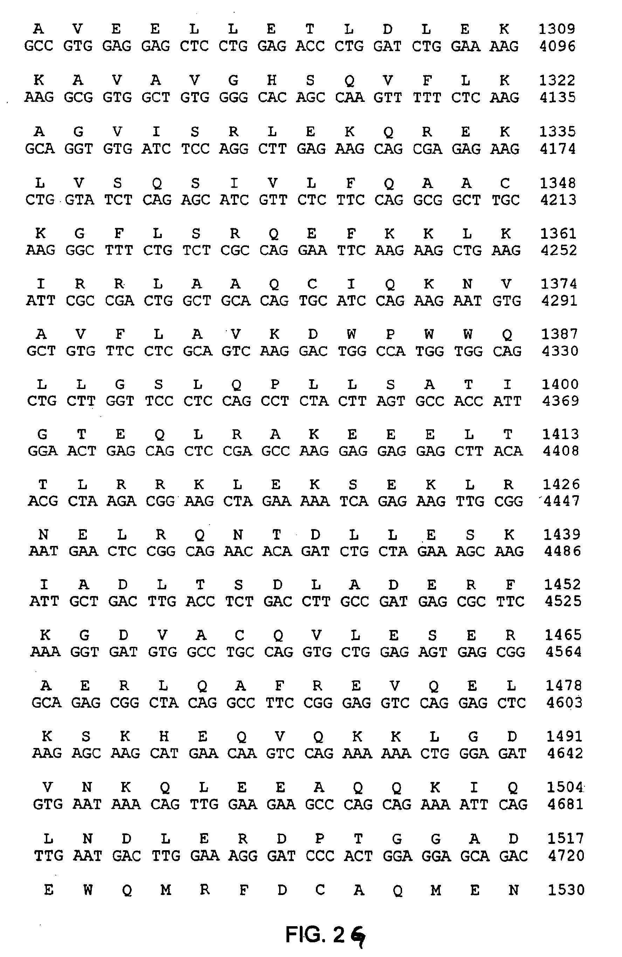

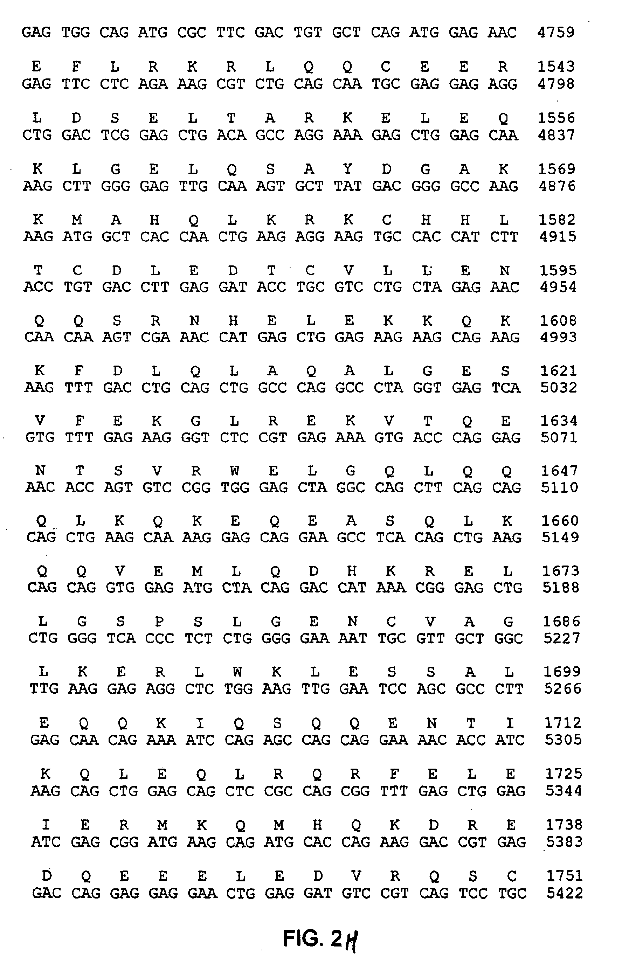

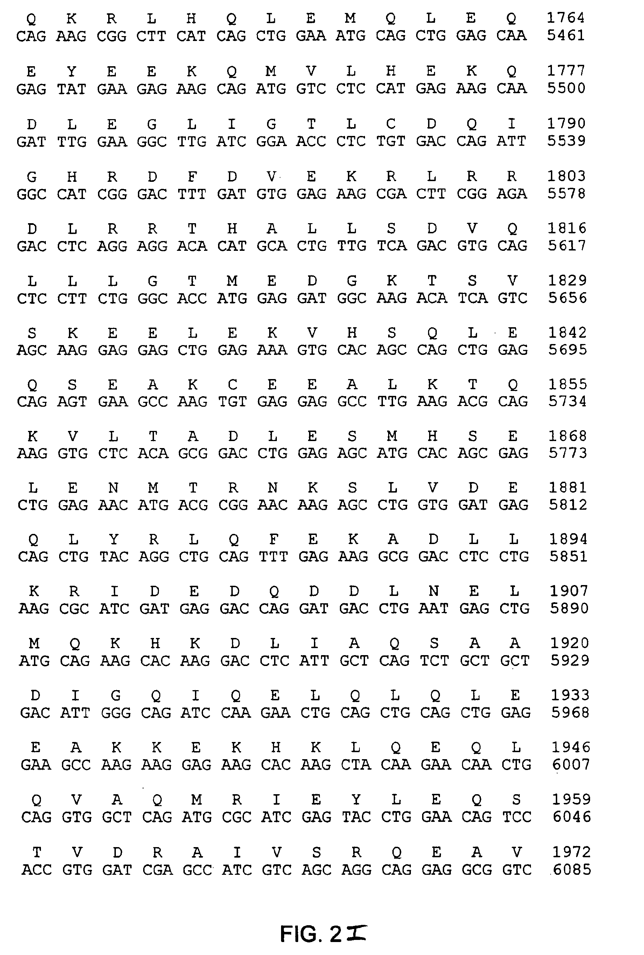

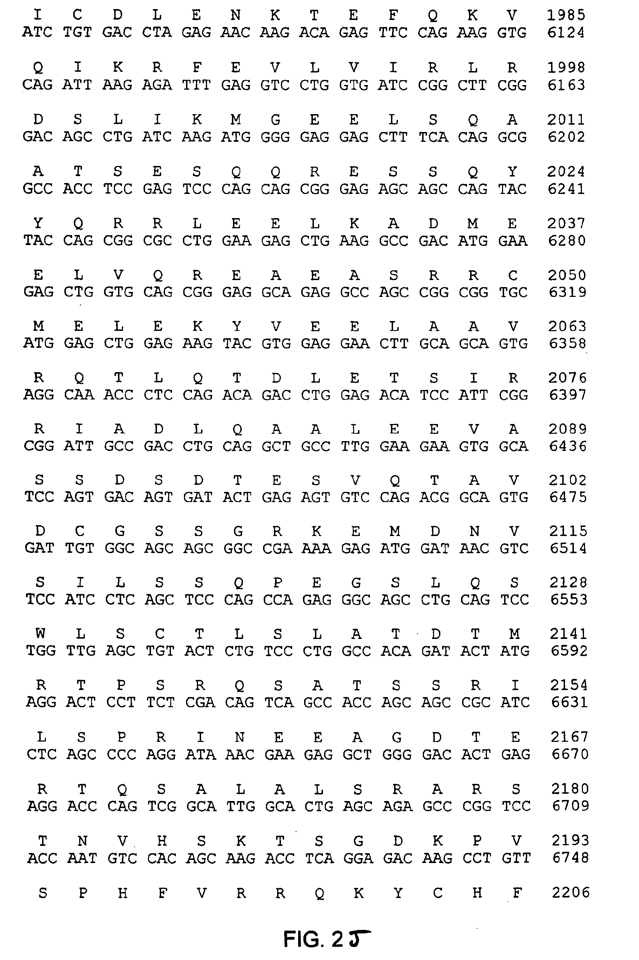

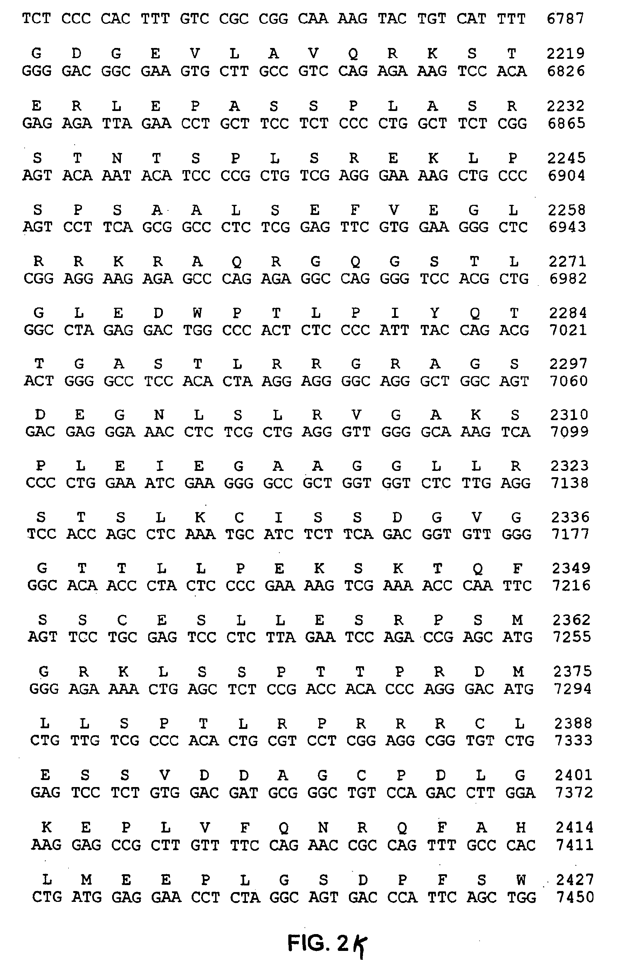

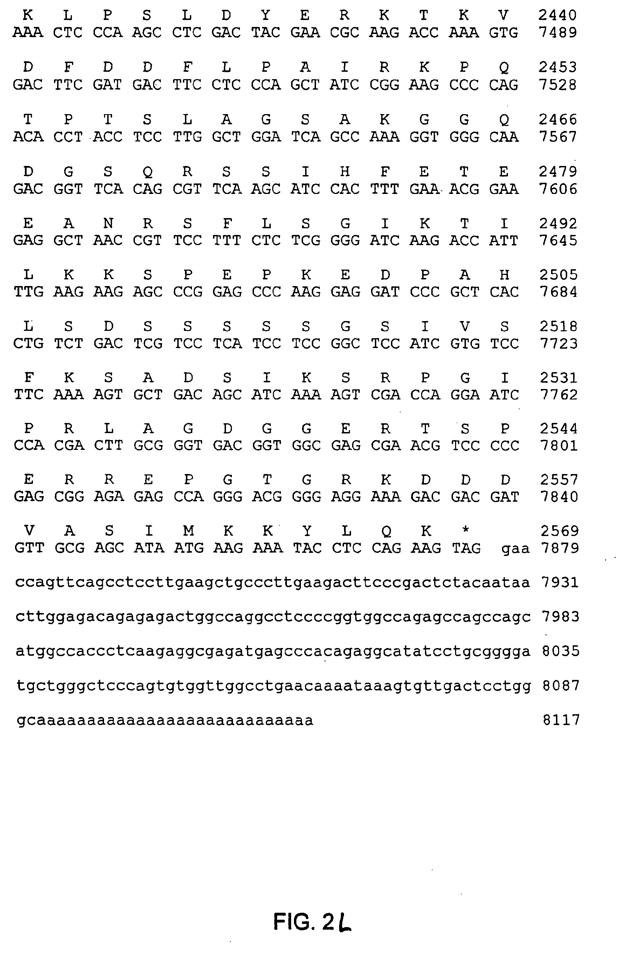

In one embodiment, the invention encompasses a polypeptide comprising the amino acid sequence of SEQ ID NO:2, as shown in FIG. 2.

The invention also encompasses hGDMLP-1 variants. A preferred hGDMLP-1 variant is one having at least 65%, 70%, 80%, 85%, 90%, 95%, 96%, 97%, 98% or even 99%, amino acid sequence identity to the hGDMLP-1 amino acid sequence, retaining at least one biological, immunological, or other functional characteristic or activity of hGDMLP-1. A preferred hGDMLP-1 variant is one having at least 95% amino acid sequence identity to SEQ ID NO:2.

The invention also encompasses polynucleotides which encode hGDMLP-1. Accordingly, any nucleic acid sequence which encodes the amino acid sequence of hGDMLP-1 can be used to produce recombinant molecules which express hGDMLP-1. In a particular embodiment, the invention encompasses the polynucleotide comprising the nucleic acid sequence of SEQ ID NO:1 as shown in FIG. 2.

It will be appreciated by those skilled in the art that as a result of the degeneracy of the genetic code, a multitude of nucleotide sequences encoding hGDMLP-1, some bearing minimal homology to the nucleotide sequences of any known and naturally occurring gene, may be produced. Thus, the invention contemplates each and every possible variation of nucleotide sequence that could be made by selecting combinations based on possible codon choices. These combinations are made in accordance with the standard triplet genetic code as applied to the nucleotide sequence of naturally occurring hGDMLP-1, and all such variations are to be considered as being specifically disclosed.

Although nucleotide sequences which encode hGDMLP-1 and its variants are preferably capable of hybridizing to the nucleotide sequence of the naturally occurring hGDMLP-1 under appropriately selected conditions of stringency, it may be advantageous to produce nucleotide sequences encoding hGDMLP-1 or its derivatives possessing a substantially different codon usage. Codons may be selected to increase the rate at which expression of the peptide occurs in a particular prokaryotic or eukaryotic host in accordance with the frequency with which particular codons are utilized by the host. Other reasons for substantially altering the nucleotide sequence encoding hGDMLP-1 and its derivatives without altering the encoded amino acid sequences include the production of RNA transcripts having more desirable properties, such as a greater half-life, than transcripts produced from the naturally occurring sequence.

The invention also encompasses production of DNA sequences, or fragments thereof, which encode hGDMLP-1 and its derivatives, entirely by synthetic chemistry. After production, the synthetic sequence may be inserted into any of the many available expression vectors and cell systems using reagents that are well known in the art. Moreover, synthetic chemistry may be used to introduce mutations into a sequence encoding hGDMLP-1 or any fragment thereof.

Also encompassed by the invention are polynucleotide sequences that are capable of hybridizing to the claimed nucleotide sequences, and in particular, those shown in SEQ ID NO:1, under various conditions of stringency as taught in Wahl, G. M. and S. L. Berger (1987; Methods Enzymol. 152:399-407) and Kimmel, A. R. (1987; Methods Enzymol. 152:507-511).

Methods for DNA sequencing which are well known and generally available in the art and may be used to practice any of the embodiments of the invention. The methods may employ such enzymes as the Klenow fragment of DNA polymerase I, SEQUENASE (U.S. Biochemical Corp, Cleveland, Ohio), Taq polymerase (Perkin Elmer), thermostable T7 polymerase (Amersham, Chicago, Ill.), or combinations of polymerases and proofreading exonucleases such as those found in the ELONGASE Amplification System marketed by Gibco/BRL (Gaithersburg, Md.). Preferably, the process is automated with machines such as the Hamilton Micro Lab 2200 (Hamilton, Reno, Nev.), Peltier Thermal Cycler (PTC200; MJ Research, Watertown, Mass.) and the MegaBACE™ DNA Sequencers (Molecular Dynamics™).

The nucleic acid sequences encoding hGDMLP-1 may be extended utilizing a partial nucleotide sequence and employing various methods known in the art to detect upstream sequences such as promoters and regulatory elements. For example, one method which may be employed, "restriction-site" PCR, uses universal primers to retrieve unknown sequence adjacent to a known locus (Sarkar, G. (1993) PCR Methods Applic. 2:318-322). In particular, genomic DNA is first amplified in the presence of primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR may also be used to amplify or extend sequences using divergent primers based on a known region (Triglia, T. et al. (1988) Nucleic Acids Res. 16:8186). The primers may be designed using commercially available software such as OLIGO 4.06 Primer Analysis software (National Biosciences Inc., Plymouth, Minn.), or another appropriate program, to be 22-30 nucleotides in length, to have a GC content of 50% or more, and to anneal to the target sequence at temperatures about 68°C-72°C. The method uses several restriction enzymes to generate a suitable fragment in the known region of a gene. The fragment is then circularized by intramolecular ligation and used as a PCR template.

Another method which may be used is capture PCR which involves PCR amplification of DNA fragments adjacent to a known sequence in human and yeast artificial chromosome DNA (Lagerstrom, M. et al. (1991) PCR Methods Applic. 1:111-119). In this method, multiple restriction enzyme digestions and ligations may also be used to place an engineered double-stranded sequence into an unknown fragment of the DNA molecule before performing PCR.

Another method which may be used to retrieve unknown sequences is that of Parker, J. D. et al. (1991; Nucleic Acids Res. 19:3055-3060). Additionally, one may use PCR, nested primers, and PROMOTERFINDER libraries (Clontech, Palo Alto, Calif.) to walk genomic DNA. This process avoids the need to screen libraries and is useful in finding intron-exon junctions.

When screening for full-length cDNAs, it is preferable to use libraries that have been size-selected to include larger cDNAs. Also, random-primed libraries are preferable, in that they will contain more sequences which contain the 5' regions of genes. Use of a randomly primed library may be especially preferable for situations in which an oligo d(T) library does not yield a full-length cDNA. Genomic libraries may be useful for extension of sequence into 5' non-transcribed regulatory regions.

Capillary electrophoresis systems which are commercially available may be used to analyze the size or confirm the nucleotide sequence of sequencing or PCR products. In particular, capillary sequencing may employ flowable polymers for electrophoretic separation, four different fluorescent dyes (one for each nucleotide) which are laser activated, and detection of the emitted wavelengths by a charge coupled device camera. Output/light intensity may be converted to electrical signal using appropriate software (e.g. GENOTYPER and SEQUENCE NAVIGATOR, Perkin Elmer) and the entire process from loading of samples to computer analysis and electronic data display may be computer controlled. Capillary electrophoresis is especially preferable for the sequencing of small pieces of DNA which might be present in limited amounts in a particular sample.

In another embodiment of the invention, polynucleotide sequences or fragments thereof which encode hGDMLP-1 may be used in recombinant DNA molecules to direct expression of hGDMLP-1, fragments or functional equivalents thereof, in appropriate host cells. Due to the inherent degeneracy of the genetic code, other DNA sequences which encode substantially the same or a functionally equivalent amino acid sequence may be produced, and these sequences may be used to clone and express hGDMLP-1.

As will be understood by those of skill in the art, it may be advantageous to produce hGDMLP-1-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences of the present invention can be engineered using methods generally known in the art in order to alter hGDMLP-1 encoding sequences for a variety of reasons, including but not limited to, alterations which modify the cloning, processing, and/or expression of the gene product. DNA shuffling by random fragmentation and PCR reassembly of gene fragments and synthetic oligonucleotides may be used to engineer the nucleotide sequences. For example, site-directed mutagenesis may be used to insert new restriction sites, alter glycosylation patterns, change codon preference, produce splice variants, introduce mutations, and so forth.

In another embodiment of the invention, natural, modified, or recombinant nucleic acid sequences encoding hGDMLP-1 may be ligated to a heterologous sequence to encode a fusion protein. For example, to screen peptide libraries for inhibitors of hGDMLP-1 activity, it may be useful to encode a chimeric hGDMLP-1 protein that can be recognized by a commercially available antibody. A fusion protein may also be engineered to contain a cleavage site located between the hGDMLP-1 encoding sequence and the heterologous protein sequence, so that hGDMLP-1 may be cleaved and purified away from the heterologous moiety.

In another embodiment, sequences encoding hGDMLP-1 may be synthesized, in whole or in part, using chemical methods well known in the art (see Caruthers, M. H. et al. (1980) Nucl. Acids Res. Symp. Ser. 215-223, Horn, T. et al. (1980) Nucl. Acids Res. Symp. Ser. 225-232). Alternatively, the protein itself may be produced using chemical methods to synthesize the amino acid sequence of hGDMLP-1, or a fragment thereof. For example, peptide synthesis can be performed using various solid-phase techniques (Roberge, J. Y. et al. (1995) Science 269:202-204) and automated synthesis may be achieved, for example, using the ABI 431A Peptide Synthesizer (Perkin Elmer).

The newly synthesized peptide may be substantially purified by preparative high performance liquid chromatography (e.g., Creighton, T. (1983) Proteins, Structures and Molecular Principles, WH Freeman and Co., New York, N.Y.). The composition of the synthetic peptides may be confirmed by amino acid analysis or sequencing (e.g., the Edman degradation procedure; Creighton, supra). Additionally, the amino acid sequence of hGDMLP-1, or any part thereof, may be altered during direct synthesis and/or combined using chemical methods with sequences from other proteins, or any part thereof, to produce a variant polypeptide.

In order to express a biologically active hGDMLP-1, the nucleotide sequences encoding hGDMLP-1 or functional equivalents, may be inserted into appropriate expression vector, i.e., a vector which contains the necessary elements for the transcription and translation of the inserted coding sequence.

Methods which are well known to those skilled in the art may be used to construct expression vectors containing sequences encoding hGDMLP-1 and appropriate transcriptional and translational control elements. These methods include in vitro recombinant DNA techniques, synthetic techniques, and in vivo genetic recombination. Such techniques are described in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York, N.Y.

A variety of expression vector/host systems may be utilized to contain and express sequences encoding hGDMLP-1. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems. The invention is not limited by the host cell employed.

The "control elements" or "regulatory sequences" are those non-translated regions of the vector, e.g. enhancers, promoters, 5' and 3' untranslated regions, which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the BLUESCRIPT phagemid (Stratagene, LaJolla, Calif.) or PSPORTI plasmid (Gibco BRL) and the like may be used. The baculovirus polyhedrin promoter may be used in insect cells. Promoters or enhancers derived from the genomes of plant cells (e.g., heat shock, RUBISCO; and storage protein genes) or from plant viruses (e.g., viral promoters or leader sequences) may be cloned into the vector. In mammalian cell systems, promoters from mammalian genes or from mammalian viruses are preferable. If it is necessary to generate a cell line that contains multiple copies of the sequence encoding hGDMLP-1, vectors based on SV40 or EBV may be used with an appropriate selectable marker.

In bacterial systems, a number of expression vectors may be selected depending upon the use intended for hGDMLP-1. For example, when large quantities of hGDMLP-1 are needed for the induction of antibodies, vectors which direct high level expression of fusion proteins that are readily purified may be used. Such vectors include, but are not limited to, the multifunctional E. coli cloning and expression vectors such as BLUESCRIPT (Stratagene), in which the sequence encoding hGDMLP-1 may be ligated into the vector in frame with sequences for the amino-terminal Met and the subsequent 7 residues of β-galactosidase so that a hybrid protein is produced; pIN vectors (Van Heeke, G. and S. M. Schuster (1989) J. Biol. Chem. 264:5503-5509); and the like. pGEX vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor Xa protease cleavage sites so that the cloned polypeptide of interest can be released from the GST moiety at will.

In the yeast, Saccharomyces cerevisiae, a number of vectors containing constitutive or inducible promoters such as alpha factor, alcohol oxidase, and PGH may be used. For reviews, see Ausubel et al. (supra) and Grant et al. (1987) Methods Enzymol. 153:516-544.

In cases where plant expression vectors are used, the expression of sequences encoding hGDMLP-1 may be driven by any of a number of promoters. For example, viral promoters such as the 35S and 19S promoters of CaMV may be used alone or in combination with the omega leader sequence from TMV (Takamatsu, N. (1987) EMBO J. 6:307-311). Alternatively, plant promoters such as the small subunit of RUBISCO or heat shock promoters may be used (Coruzzi, G. et al. (1984) EMBO J. 3:1671-1680; Broglie, R. et al. (1984) Science 224:838-843; and Winter, J. et al. (1991) Results Probl. Cell Differ. 17:85-105). These constructs can be introduced into plant cells by direct DNA transformation or pathogen-mediated transfection. Such techniques are described in a number of generally available reviews (see, for example, Hobbs, S. or Murry, L. E. in McGraw Hill Yearbook of Science and Technology (1992) McGraw Hill, New York, N. Y.; pp. 191-196).

An insect system may also be used to express hGDMLP-1. For example, in one such system, Autographa californica nuclear polyhedrosis virus (AcNPV) is used as a vector to express foreign genes in Spodoptera frugiperda cells or in Trichoplusia larvae. The sequences encoding hGDMLP-1 may be cloned into a non-essential region of the virus, such as the polyhedrin gene, and placed under control of the polyhedrin promoter. Successful insertion of hGDMLP-1 will render the polyhedrin gene inactive and produce recombinant virus lacking coat protein. The recombinant viruses may then be used to infect, for example, S. frugiperda cells or Trichoplusia larvae in which hGDMLP-1 may be expressed (Engelhard, E. K. et al. (1994) Proc. Nat. Acad. Sci. 91:3224-3227).

In mammalian host cells, a number of viral-based expression systems may be utilized. In cases where an adenovirus is used as an expression vector, sequences encoding hGDMLP-1 may be ligated into an adenovirus transcription/translation complex consisting of the late promoter and tripartite leader sequence. Insertion in a non-essential E1 or E3 region of the viral genome may be used to obtain a viable virus which is capable of expressing hGDMLP-1 in infected host cells (Logan, J. and Shenk, T. (1984) Proc. Natl. Acad. Sci. 81:3655-3659). In addition, transcription enhancers, such as the Rous sarcoma virus (RSV) enhancer, may be used to increase expression in mammalian host cells.

Human artificial chromosomes (HACs) may also be employed to deliver larger fragments of DNA than can be contained and expressed in a plasmid. HACs of 6M to 10M basepairs are constructed and delivered via conventional delivery methods (liposomes, polycationic amino polymers, or vesicles) for therapeutic purposes.

Specific initiation signals may also be used to achieve more efficient translation of sequences encoding hGDMLP-1. Such signals include the ATG initiation codon and adjacent sequences. In cases where sequences encoding hGDMLP-1, its initiation codon, and upstream sequences are inserted into the appropriate expression vector, no additional transcriptional or translational control signals may be needed. However, in cases where only coding sequence, or a fragment thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125- 162).

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding and/or function. Different host cells which have specific cellular machinery and characteristic mechanisms for post-translational activities (e.g., CHO, HeLa, MDCK, HEK293, and WI38), are available from the American Type Culture Collection (ATCC; Bethesda, Md.) and may be chosen to ensure the correct modification and processing of the foreign protein.

For long-term, high-yield production of recombinant proteins, stable expression is preferred. For example, cell lines which stably express hGDMLP-1 may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be expanded using tissue culture techniques appropriate to the cell type.

Any number of selection systems may be used to recover transformed cell lines. These include, but are not limited to, the herpes simplex virus thymidine kinase (Wigler, M. et al. (1977) Cell 11:223-32) and adenine phosphoribosyltransferase (Lowy, I. et al. (1980) Cell 22:817-23) genes which can be employed in tk⁻ or aprt⁻ cells, respectively. Also, antimetabolite, antibiotic or herbicide resistance can be used as the basis for selection; for example, dhfr which confers resistance to methotrexate (Wigler, M. et al. (1980) Proc. Natl. Acad. Sci. 77:3567-70); npt, which confers resistance to the aminoglycosides, neomycin and G-418 (Colbere-Garapin, F. et al (1981) J. Mol. Biol. 150:1-14); and als or pat, which confer resistance to chlorsulfuron and phosphinotricin acetyltransferase, respectively (Murry, supra). Additional selectable genes have been described, for example, trpB, which allows cells to utilize indole in place of tryptophan, or hisD, which allows cells to utilize histinol in place of histidine (Hartman, S. C. and R. C. Mulligan (1988) Proc. Natl. Acad. Sci. 85:8047- 51). Recently, the use of visible markers has gained popularity with such markers as anthocyanins, β-glucuronidase and its substrate GUS, and luciferase and its substrate luciferin, being widely used not only to identify transformants, but also to quantify the amount of transient or stable protein expression attributable to a specific vector system (Rhodes, C. A. et al. (1995) Methods Mol. Biol. 55:121-131).

Although the presence/absence of marker gene expression suggests that the gene of interest is also present, its presence and expression may need to be confirmed. For example, if the sequence encoding hGDMLP-1 is inserted within a marker gene sequence, transformed cells containing sequences encoding hGDMLP-1 can be identified by the absence of marker gene function. Alternatively, a marker gene can be placed in tandem with a sequence encoding hGDMLP-1 under the control of a single promoter. Expression of the marker gene in response to induction or selection usually indicates expression of the tandem gene as well.

Alternatively, host cells which contain the nucleic acid sequence encoding hGDMLP-1 and express hGDMLP-1 may be identified by a variety of procedures known to those of skill in the art. These procedures include, but are not limited to, DNA-DNA or DNA-RNA hybridizations and protein bioassay or immunoassay techniques which include membrane, solution, or chip based technologies for the detection and/or quantification of nucleic acid or protein.

The presence of polynucleotide sequences encoding hGDMLP-1 can be detected by DNA-DNA or DNA-RNA hybridization or amplification using probes or fragments or fragments of polynucleotides encoding hGDMLP-1. Nucleic acid amplification based assays involve the use of oligonucleotides or oligomers based on the sequences encoding hGDMLP-1 to detect transformants containing DNA or RNA encoding hGDMLP-1.

A variety of protocols for detecting and measuring the expression of hGDMLP-1, using either polyclonal or monoclonal antibodies specific for the protein are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on hGDMLP-1 is preferred, but a competitive binding assay may be employed. These and other assays are described, among other places, in Hampton, R. et al. (1990; Serological Methods, a Laboratory Manual, APS Press, St Paul, Minn.) and Maddox, D. E. et al. (1983; J. Exp. Med. 158:1211-1216).

A wide variety of labels and conjugation techniques are known by those skilled in the art and may be used in various nucleic acid and amino acid assays. Means for producing labeled hybridization or PCR probes for detecting sequences related to polynucleotides encoding hGDMLP-1 include oligolabeling, nick translation, end-labeling or PCR amplification using a labeled nucleotide. Alternatively, the sequences encoding hGDMLP-1, or any fragments thereof may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits (Amersham Pharmacia Biotech, Uppsala, Sweden; Promega, Madison, Wisconsin; and U.S. Biochemical Corp., Cleveland, Ohio). Suitable reporter molecules or labels, which may be used for ease of detection, include radionuclides, enzymes, fluorescent, chemiluminescent, or chromogenic agents as well as substrates, cofactors, inhibitors, magnetic particles, and the like.

Host cells transformed with nucleotide sequences encoding hGDMLP-1 may be cultured under conditions suitable for the expression and recovery of the protein from cell culture. The protein produced by a transformed cell may be secreted or contained intracellularly depending on the sequence and/or the vector used. As will be understood by those of skill in the art, expression vectors containing polynucleotides which encode hGDMLP-1 may be designed to contain signal sequences which direct secretion of hGDMLP-1 through a prokaryotic or eukaryotic cell membrane. Other constructions may be used to join sequences encoding hGDMLP-1 to nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). The inclusion of cleavable linker sequences such as those specific for Factor Xa or enterokinase (Invitrogen, San Diego, Calif.) between the purification domain and hGDMLP-1 may be used to facilitate purification. One such expression vector provides for expression of a fusion protein containing hGDMLP-1 and a nucleic acid encoding 6 histidine residues preceding a thioredoxin or an enterokinase cleavage site. The histidine residues facilitate purification on IMAC (immobilized metal ion affinity chromatography) as described in Porath, J. et al. (1992, Prot. Exp. Purif. 3: 263-281) while the enterokinase cleavage site provides a means for purifying hGDMLP-1 from the fusion protein. A discussion of vectors which contain fusion proteins is provided in Kroll, D. J. et al. (1993; DNA Cell Biol. 12:441-453).

In addition to recombinant production, fragments of hGDMLP-1 may be produced by direct peptide synthesis using solid-phase techniques (Merrifield J. (1963) J. Am. Chem. Soc. 85:2149-2154). Protein synthesis may be performed using manual techniques or by automation. Automated synthesis may be achieved, for example, using Applied Biosystems 431A Peptide Synthesizer (Perkin Elmer). Various fragments of hGDMLP-1 may be chemically synthesized separately and combined using chemical methods to produce the full length molecule.

### Therapeutics

In one embodiment, hGDMLP-1 or a fragment or derivative thereof may be administered to a subject to treat or prevent a heart or skeletal muscle disorder.

In another embodiment, a vector capable of expressing hGDMLP-1, or a fragment or a derivative thereof, may also be administered to a subject to treat or prevent a heart or skeletal muscle disorder.

In another embodiment, an agonist which modulates the activity of hGDMLP-1 may also be administered to a subject to treat or prevent a heart or skeletal muscle disorder.

In another embodiment, an antagonist of hGDMLP-1 may be administered to a subject to treat or prevent a heart or skeletal muscle disorder.

In another embodiment, an antibody which specifically binds hGDMLP-1 may be used directly as an antagonist or indirectly as a targeting or delivery mechanism for bringing a pharmaceutical agent to cells or tissue which express hGDMLP-1.

In another embodiment, a vector expressing the complement of the polynucleotide encoding hGDMLP-1 may be administered to a subject to treat or prevent a heart or skeletal muscle disorder.

In other embodiments, any of the proteins, antagonists, antibodies, agonists, complementary sequences or vectors of the invention may be administered in combination with other appropriate therapeutic agents. Selection of the appropriate agents for use in combination therapy may be made by one of ordinary skill in the art, according to conventional pharmaceutical principles. The combination of therapeutic agents may act synergistically to effect the treatment or prevention of the various disorders described above. Using this approach, one may be able to achieve therapeutic efficacy with lower dosages of each agent, thus reducing the potential for adverse side effects.

An antagonist of hGDMLP-1 may be produced using methods which are generally known in the art. In particular, purified hGDMLP-1 may be used to produce antibodies or to screen libraries of pharmaceutical agents to identify those which specifically bind hGDMLP-1.

Antibodies to hGDMLP-1 may be generated using methods that are well known in the art. Such antibodies may include, but are not limited to, polyclonal, monoclonal, chimeric, single chain, Fab fragments, and fragments produced by a Fab expression library. Neutralizing antibodies, (i.e., those which inhibit dimer formation) are especially preferred for therapeutic use.

For the production of antibodies, various hosts including goats, rabbits, rats, mice, humans, and others, may be immunized by injection with hGDMLP-1 or any fragment or oligopeptide thereof which has immunogenic properties. Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, keyhole limpet hemocyanin, and dinitrophenol. Among adjuvants used in humans, BCG (bacilli Calmette- Guerin) and Corynebacterium parvum are especially preferable.

It is preferred that the oligopeptides, peptides, or fragments used to induce antibodies to hGDMLP-1 have an amino acid sequence consisting of at least five amino acids and more preferably at least 10 amino acids. It is also preferable that they are identical to a portion of the amino acid sequence of the natural protein, and they may contain the entire amino acid sequence of a small, naturally occurring molecule. Short stretches of hGDMLP-1 amino acids may be fused with those of another protein such as keyhole limpet hemocyanin and antibody produced against the chimeric molecule.

Monoclonal antibodies to hGDMLP-1 may be prepared using any technique which provides for the production of antibody molecules by continuous cell lines in culture. These include, but are not limited to, the hybridoma technique, the human B-cell hybridoma technique, and the EBV- hybridoma technique (Kohler, G. et al. (1975) Nature 256:495-497; Kozbor, D. et al. (1985) J. Immunol. Methods 81:31-42; Cote, R. J. et al. (1983) Proc. Natl. Acad. Sci. 80:2026-2030; Cole, S. P. et al. (1984) Mol. Cell Biol. 62:109-120).

In addition, techniques developed for the production of "chimeric antibodies", the splicing of mouse antibody genes to human antibody genes to obtain a molecule with appropriate antigen specificity and biological activity can be used (Morrison, S. L. et al. (1984) Proc. Natl. Acad. Sci. 81:6851-6855; Neuberger, M. S. et al. (1984) Nature 312:604-608; Takeda, S. et al. (1985) Nature 314:452-454). Alternatively, techniques described for the production of single chain antibodies may be adapted, using methods known in the art, to produce hGDMLP-1-specific single chain antibodies. Antibodies with related specificity, but of distinct idiotypic composition, may be generated by chain shuffling from random combinatorial immunoglobulin libraries (Burton D. R. (1991) Proc. Natl. Acad. Sci. 88:11120-3).

Antibodies may also be produced by inducing in vivo production in the lymphocyte population or by screening immunoglobulin libraries or panels of highly specific binding reagents as disclosed in the literature (Orlandi, R. et al. (1989) Proc. Natl. Acad. Sci. 86: 3833- 3837; Winter, G. et al. (1991) Nature 349:293-299).

Antibody fragments which contain specific binding sites for hGDMLP-1 may also be generated. For example, such fragments include, but are not limited to, the F(ab')2 fragments which can be produced by pepsin digestion of the antibody molecule and the Fab fragments which can be generated by reducing the disulfide bridges of the F(ab')2 fragments. Alternatively, Fab expression libraries may be constructed to allow rapid and easy identification of monoclonal Fab fragments with the desired specificity (Huse, W. D. et al. (1989) Science 254:1275-1281).

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between hGDMLP-1 and its specific antibody. A two-site, monoclonal-based immunoassay utilizing monoclonal antibodies reactive to two non-interfering hGDMLP-1 epitopes is preferred, but a competitive binding assay may also be employed (Maddox, supra).

In another embodiment of the invention, the polynucleotides encoding hGDMLP-1, or any fragment or complement thereof, may be used for therapeutic purposes. In one aspect, the complement of the polynucleotide encoding hGDMLP-1 may be used in situations in which it would be desirable to block the transcription of the mRNA. In particular, cells may be transformed with sequences complementary to polynucleotides encoding hGDMLP-1. Thus, complementary molecules or fragments may be used to modulate hGDMLP-1 activity, or to achieve regulation of gene function. Such technology is now well known in the art, and sense or antisense oligonucleotides or larger fragments, can be designed from various locations along the coding or control regions of sequences encoding hGDMLP-1.

Expression vectors derived from retroviruses, adenovirus, herpes or vaccinia viruses, or from various bacterial plasmids may be used for delivery of nucleotide sequences to the targeted organ, tissue or cell population. Methods which are well known to those skilled in the art can be used to construct vectors which will express nucleic acid sequence which is complementary to the polynucleotides of the gene encoding hGDMLP-1. These techniques are described both in Sambrook et al. (supra) and in Ausubel et al. (supra).

Genes encoding hGDMLP-1 can be turned off by transforming a cell or tissue with expression vectors which express high levels of a polynucleotide or fragment thereof which encodes hGDMLP-1. Such constructs may be used to introduce untranslatable sense or antisense sequences into a cell. Even in the absence of integration into the DNA, such vectors may continue to transcribe RNA molecules until they are disabled by endogenous nucleases. Transient expression may last for a month or more with a non-replicating vector and even longer if appropriate replication elements are part of the vector system.

As mentioned above, modifications of gene expression can be obtained by designing complementary sequences or antisense molecules (DNA, RNA, or PNA) to the control, 5' or regulatory regions of the gene encoding hGDMLP-1 (signal sequence, promoters, enhancers, and introns). Oligonucleotides derived from the transcription initiation site, e.g., between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or regulatory molecules. Recent therapeutic advances using triplex DNA have been described in the literature (Gee, J. E. et al. (1994) In: Huber, B. E. and B. I. Carr, Molecular and Immunologic Approaches, Futura Publishing Co., Mt. Kisco, N.Y.). The complementary sequence or antisense molecule may also be designed to block translation of mRNA by preventing the transcript from binding to ribosomes.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. Examples which may be used include engineered hammerhead motif ribozyme molecules that can specifically and efficiently catalyze endonucleolytic cleavage of sequences encoding hGDMLP-1.

Specific ribozyme cleavage sites within any potential RNA target are initially identified by scanning the target molecule for ribozyme cleavage sites which include the following sequences: GUA, GUU, and GUC. Once identified, short RNA sequences of between 15 and 20 ribonucleotides corresponding to the region of the target gene containing the cleavage site may be evaluated for secondary structural features which may render the oligonucleotide inoperable. The suitability of candidate targets may also be evaluated by testing accessibility to hybridization with complementary oligonucleotides using ribonuclease protection assays.

Complementary ribonucleic acid molecules and ribozymes of the invention may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences encoding hGDMLP-1. Such DNA sequences may be incorporated into a wide variety of vectors with suitable RNA polymerase promoters such as T7 or SP6. Alternatively, these cDNA constructs that synthesize complementary RNA constitutively or inducibly can be introduced into cell lines, cells, or tissues.

RNA molecules may be modified to increase intracellular stability and half-life. Possible modifications include, but are not limited to, the addition of flanking sequences at the 5' and/or 3' ends of the molecule or the use of phosphorothioate or 2' O-methyl rather than phosphodiesterase linkages within the backbone of the molecule. This concept is inherent in the production of PNAs and can be extended in all of these molecules by the inclusion of nontraditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection, by liposome injections or polycationic amino polymers (Goldman, C. K. et al. (1997) Nature Biotechnology 15:462-66; incorporated herein by reference) may be achieved using methods which are well known in the art.

Any of the therapeutic methods described above may be applied to any subject in need of such therapy, including, for example, mammals such as dogs, cats, cows, horses, rabbits, monkeys, and most preferably, humans.

An additional embodiment of the invention relates to the administration of a pharmaceutical composition, in conjunction with a pharmaceutically acceptable carrier, for any of the therapeutic effects discussed above. Such pharmaceutical compositions may consist of hGDMLP-1, antibodies to hGDMLP-1, mimetics, agonists, antagonists, or inhibitors of hGDMLP-1. The compositions may be administered alone or in combination with at least one other agent, such as stabilizing compound, which may be administered in any sterile, biocompatible pharmaceutical carrier, including, but not limited to, saline, buffered saline, dextrose, and water. The compositions may be administered to a patient alone, or in combination with other agents, drugs or hormones.

The pharmaceutical compositions utilized in this invention may be administered by any number of routes including, but not limited to, oral, intravenous, intramuscular, intra-arterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations which can be used orally include push- fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Pharmaceutical formulations suitable for parenteral administration may be formulated in aqueous solutions, preferably in physiologically compatible buffers such as Hanks' solution, Ringer's solution, or physiologically buffered saline. Aqueous injection suspensions may contain substances which increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers may also be used for delivery. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

The pharmaceutical compositions of the present invention may be manufactured in a manner that is known in the art, e.g., by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation may be a lyophilized powder which may contain any or all of the following: 1-50 mM histidine, 0.1%-2% sucrose, and 2-7% mannitol, at a pH range of 4.5 to 5.5, that is combined with buffer prior to use.

After pharmaceutical compositions have been prepared, they can be placed in an appropriate container and labeled for treatment of an indicated condition. For administration of hGDMLP-1, such labeling would include amount, frequency, and method of administration.

Pharmaceutical compositions suitable for use in the invention include compositions wherein the active ingredients are contained in an effective amount to achieve the intended purpose. The determination of an effective dose is well within the capability of those skilled in the art.

For any compound, the therapeutically effective dose can be estimated initially either in cell culture assays, e.g., of neoplastic cells, or in animal models, usually mice, rabbits, dogs, or pigs. The animal model may also be used to determine the appropriate concentration range and route of administration. Such information can then be used to determine useful doses and routes for administration in humans.

A therapeutically effective dose refers to that amount of active ingredient, for example hGDMLP-1 or fragments thereof, antibodies of hGDMLP-1, agonists, antagonists or inhibitors of hGDMLP-1, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active moiety or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

### Diagnostics

In another embodiment, antibodies which specifically bind hGDMLP-1 may be used for the diagnosis of conditions or diseases characterized by expression of hGDMLP-1, or in assays to monitor patients being treated with hGDMLP-1, agonists, antagonists or inhibitors. The antibodies useful for diagnostic purposes may be prepared in the same manner as those described above for therapeutics. Diagnostic assays for hGDMLP-1 include methods which utilize the antibody and a label to detect hGDMLP-1 in human body fluids or extracts of cells or tissues. The antibodies may be used with or without modification, and may be labeled by joining them, either covalently or non-covalently, with a reporter molecule. A wide variety of reporter molecules which are known in the art may be used, several of which are described above.

A variety of protocols including ELISA, RIA, and FACS for measuring hGDMLP-1 are known in the art and provide a basis for diagnosing altered or abnormal levels of hGDMLP-1 expression. Normal or standard values for hGDMLP-1 expression are established by combining body fluids or cell extracts taken from normal mammalian subjects, preferably human, with antibody to hGDMLP-1 under conditions suitable for complex formation. The amount of standard complex formation may be quantified by various methods, but preferably by photometric means. Quantities of hGDMLP-1 expressed in subject, control, and disease samples from biopsied tissues are compared with the standard values. Deviation between standard and subject values establishes the parameters for diagnosing disease.

In another embodiment of the invention, the polynucleotides encoding hGDMLP-1 may be used for diagnostic purposes. The polynucleotides which may be used include oligonucleotide sequences, complementary RNA and DNA molecules, and PNAs. The polynucleotides may be used to detect and quantitate gene expression in biopsied tissues in which expression of hGDMLP-1 may be correlated with disease. The diagnostic assay may be used to distinguish between absence, presence, and excess expression of hGDMLP-1, and to monitor regulation of hGDMLP-1 levels during therapeutic intervention.

In one aspect, hybridization with PCR probes which are capable of detecting polynucleotide sequences, including genomic sequences, encoding hGDMLP-1 or closely related molecules, may be used to identify nucleic acid sequences which encode hGDMLP-1. The specificity of the probe, whether it is made from a highly specific region, e.g., 10 unique nucleotides in the 5' regulatory region, or a less specific region, e.g., especially in the 3' coding region, and the stringency of the hybridization or amplification (maximal, high, intermediate, or low) will determine whether the probe identifies only naturally occurring sequences encoding hGDMLP-1, alleles, or related sequences.

Probes may also be used for the detection of related sequences, and should preferably contain at least 50% of the nucleotides from any of the hGDMLP-1 encoding sequences. The hybridization probes of the subject invention may be DNA or RNA and derived from the nucleotide sequence of SEQ ID NO:1, or from genomic sequence including promoter, enhancer elements, and introns of the naturally occurring hGDMLP-1 (SEQ ID NO:3).

Means for producing specific hybridization probes for DNAs encoding hGDMLP-1 include the cloning of nucleic acid sequences encoding hGDMLP-1 or hGDMLP-1 derivatives into vectors for the production of mRNA probes. Such vectors are known in the art, commercially available, and may be used to synthesize RNA probes in vitro by means of the addition of the appropriate RNA polymerases and the appropriate labeled nucleotides. Hybridization probes may be labeled by a variety of reporter groups, for example, radionuclides such as 32P or 35S, or enzymatic labels, such as alkaline phosphatase coupled to the probe via avidin/biotin coupling systems, and the like.

Polynucleotide sequences encoding hGDMLP-1 may be used for the diagnosis of a disorder associated with expression of hGDMLP-1, in particular heart and skeletal muscle disorders.

The polynucleotide sequences encoding hGDMLP-1 may be used in Southern or northern analysis, dot blot, or other membrane-based technologies; in PCR technologies; or in dipstick, pin, ELISA assays or microarrays utilizing fluids or tissues from patient biopsies to detect altered hGDMLP-1 expression. Such qualitative or quantitative methods are well known in the art.

The nucleotide sequences encoding hGDMLP-1 may be labeled by standard methods, and added to a fluid or tissue sample from a patient under conditions suitable for the formation of hybridization complexes. After a suitable incubation period, the sample is washed and the signal is quantitated and compared with a standard value. If the amount of signal in the biopsied or extracted sample is significantly altered from that of a comparable control sample, the nucleotide sequences have hybridized with nucleotide sequences in the sample, and the presence of altered levels of nucleotide sequences encoding hGDMLP-1 in the sample indicates the presence of the associated disease. Such assays may also be used to evaluate the efficacy of a particular therapeutic treatment regimen in animal studies, in clinical trials, or in monitoring the treatment of an individual patient.

In order to provide a basis for the diagnosis of disease associated with expression of hGDMLP-1, a normal or standard profile for expression is established. This may be accomplished by combining body fluids or cell extracts taken from normal subjects, either animal or human, with a sequence, or a fragment thereof, which encodes hGDMLP-1, under conditions suitable for hybridization or amplification. Standard hybridization may be quantified by comparing the values obtained from normal subjects with those from an experiment where a known amount of a substantially purified polynucleotide is used. Standard values obtained from normal samples may be compared with values obtained from samples from patients who are symptomatic for disease. Deviation between standard and subject values is used to establish the presence of disease.

Once disease is established and a treatment protocol is initiated, hybridization assays may be repeated on a regular basis to evaluate whether the level of expression in the patient begins to approximate that which is observed in the normal patient. The results obtained from successive assays may be used to show the efficacy of treatment over a period ranging from several days to months.

With respect to cancer, the presence of a relatively high amount of transcript in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Additional diagnostic uses for oligonucleotides designed from the sequences encoding hGDMLP-1 may involve the use of PCR. Such oligomers may be chemically synthesized, generated enzymatically, or produced in vitro. Oligomers will preferably consist of two nucleotide sequences, one with sense orientation and another with antisense, employed under optimized conditions for identification of a specific gene or condition. The same two oligomers, nested sets of oligomers, or even a degenerate pool of oligomers may be employed under less stringent conditions for detection and/or quantitation of closely related DNA or RNA sequences.

Methods which may also be used to quantitate the expression of hGDMLP-1 include radiolabeling or biotinylating nucleotides, coamplification of a control nucleic acid, and standard curves onto which the experimental results are interpolated (Melby, P. C. et al. (1993) J. Immunol. Methods, 159:235-244; Duplaa, C. et al. (1993) Anal. Biochem. 229-236). The speed of quantitation of multiple samples may be accelerated by running the assay in an ELISA format where the oligomer of interest is presented in various dilutions and a spectrophotometric or colorimetric response gives rapid quantitation.

In further embodiments, an oligonucleotide derived from any of the polynucleotide sequences described herein may be used as a target in a microarray. The microarray can be used to monitor the expression level of large numbers of genes simultaneously (to produce a transcript image), and to identify genetic variants, mutations and polymorphisms. This information will be useful in determining gene function, understanding the genetic basis of disease, diagnosing disease, and in developing and monitoring the activity of therapeutic agents (Heller, R. et al. (1997) Proc. Natl. Acad. Sci. 94:2150-55).

In one embodiment, the microarray is prepared and used according to the methods described in PCT application WO95/11995 (Chee et al.), Lockhart, D. J. et al. (1996; Nat. Biotech. 14: 1675-1680) and Schena, M. et al. (1996; Proc. Natl. Acad. Sci. 93: 10614-10619), all of which are incorporated herein in their entirety by reference.

The microarray is preferably composed of a large number of unique, single-stranded nucleic acid sequences, usually either synthetic antisense oligonucleotides or fragments of cDNAs, fixed to a solid support. The oligonucleotides are preferably about 6-60 nucleotides in length, more preferably 15-30 nucleotides in length, and most preferably about 20-25 nucleotides in length. For a certain type of microarray, it may be preferable to use oligonucleotides which are only 7-10 nucleotides in length. The microarray may contain oligonucleotides which cover the known 5' or 3' sequence, sequential oligonucleotides which cover the full length sequence, or unique oligonucleotides selected from particular areas along the length of the sequence. Polynucleotides used in the microarray may be oligonucleotides that are specific to a gene or genes of interest in which at least a fragment of the sequence is known or that are specific to one or more unidentified cDNAs which are common to a particular cell type, developmental or disease state.

In order to produce oligonucleotides to a known sequence for a microarray, the gene of interest is examined using a computer algorithm which starts at the 5', or more preferably, at the 3' end of the nucleotide sequence. The algorithm identifies oligomers of defined length that are unique to the gene, have a GC content within a range suitable for hybridization, and lack predicted secondary structure that may interfere with hybridization. In certain situations it may be appropriate to use pairs of oligonucleotides on a microarray. The "pairs" will be identical, except for one nucleotide which preferably is located in the center of the sequence. The second oligonucleotide in the pair (mismatched by one) serves as a control. The number of oligonucleotide pairs may range from two to one million. The oligomers are synthesized at designated areas on a substrate using a light-directed chemical process. The substrate may be paper, nylon or other type of membrane, filter, chip, glass slide or any other suitable solid support.

In another aspect, an oligonucleotide may be synthesized on the surface of the substrate by using a chemical coupling procedure and an ink jet application apparatus, as described in PCT application WO95/251116 (Baldeschweiler et al.) which is incorporated herein in its entirety by reference. In another aspect, a "gridded" array analogous to a dot (or slot) blot may be used to arrange and link cDNA fragments or oligonucleotides to the surface of a substrate using a vacuum system, thermal, UV, mechanical or chemical bonding procedures. An array, such as those described above, may be produced by hand or by using available devices (slot blot or dot blot apparatus), materials (any suitable solid support), and machines (including robotic instruments), and may contain 8, 24, 96, 384, 1536 or 6144 oligonucleotides, or any other number between two and one million which lends itself to the efficient use of commercially available instrumentation.

In order to conduct sample analysis using a microarray, the RNA or DNA from a biological sample is made into hybridization probes. The mRNA is isolated, and cDNA is produced and used as a template to make antisense RNA (aRNA). The aRNA is amplified in the presence of fluorescent nucleotides, and labeled probes are incubated with the microarray so that the probe sequences hybridize to complementary oligonucleotides of the microarray. Incubation conditions are adjusted so that hybridization occurs with precise complementary matches or with various degrees of less complementarity. After removal of nonhybridized probes, a scanner is used to determine the levels and patterns of fluorescence. The scanned images are examined to determine degree of complementarity and the relative abundance of each oligonucleotide sequence on the microarray. The biological samples may be obtained from any bodily fluids (such as blood, urine, saliva, phlegm, gastric juices, etc.), cultured cells, biopsies, or other tissue preparations. A detection system may be used to measure the absence, presence, and amount of hybridization for all of the distinct sequences simultaneously. This data may be used for large scale correlation studies on the sequences, mutations, variants, or polymorphisms among samples.

In another embodiment of the invention, the nucleic acid sequences which encode hGDMLP-1 may also be used to generate hybridization probes which are useful for mapping the naturally occurring genomic sequence. The sequences may be mapped to a particular chromosome, to a specific region of a chromosome or to artificial chromosome constructions, such as human artificial chromosomes (HACs), yeast artificial chromosomes (YACs), bacterial artificial chromosomes (BACs), bacterial P1 constructions or single chromosome cDNA libraries as reviewed in Price, C. M. (1993) Blood Rev. 7:127-134, and Trask, B. J. (1991) Trends Genet. 7:149-154.

Fluorescent in situ hybridization (FISH as described in Verma et al. (1988) Human Chromosomes: A Manual of Basic Techniques, Pergamon Press, New York, N.Y.) may be correlated with other physical chromosome mapping techniques and genetic map data. Examples of genetic map data can be found in various scientific journals or at Online Mendelian Inheritance in Man (OMIM). Correlation between the location of the gene encoding hGDMLP-1 on a physical chromosomal map and a specific disease, or predisposition to a specific disease, may help delimit the region of DNA associated with that genetic disease. The nucleotide sequences of the subject invention may be used to detect differences in gene sequences between normal, carrier, or affected individuals.

In situ hybridization of chromosomal preparations and physical mapping techniques such as linkage analysis using established chromosomal markers may be used for extending genetic maps. Often the placement of a gene on the chromosome of another mammalian species, such as mouse, may reveal associated markers even if the number or arm of a particular human chromosome is not known. New sequences can be assigned to chromosomal arms, or parts thereof, by physical mapping. This provides valuable information to investigators searching for disease genes using positional cloning or other gene discovery techniques. Once the disease or syndrome has been crudely localized by genetic linkage to a particular genomic region, for example, AT to 11q22-23 (Gatti, R. A. et al. (1988) Nature 336:577-580), any sequences mapping to that area may represent associated or regulatory genes for further investigation. The nucleotide sequence of the subject invention may also be used to detect differences in the chromosomal location due to translocation, inversion, etc. among normal, carrier, or affected individuals.

In another embodiment of the invention, hGDMLP-1, its catalytic or immunogenic fragments or oligopeptides thereof, can be used for screening libraries of compounds in any of a variety of drug screening techniques. The fragment employed in such screening may be free in solution, affixed to a solid support, borne on a cell surface, or located intracellularly. The formation of binding complexes, between hGDMLP-1 and the agent being tested, may be measured.

Another technique for drug screening which may be used provides for high throughput screening of compounds having suitable binding affinity to the protein of interest as described in published PCT application WO84/03564. In this method, as applied to hGDMLP-1 large numbers of different small test compounds are synthesized on a solid substrate, such as plastic pins or some other surface. The test compounds are reacted with hGDMLP-1, or fragments thereof, and washed. Bound hGDMLP-1 is then detected by methods well known in the art. Purified hGDMLP-1 can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable of binding hGDMLP-1 specifically compete with a test compound for binding hGDMLP-1. In this manner, the antibodies can be used to detect the presence of any peptide which shares one or more antigenic determinants with hGDMLP-1.

In additional embodiments, the nucleotide sequences which encode hGDMLP-1 may be used in any molecular biology techniques that have yet to be developed, provided the new techniques rely on properties of nucleotide sequences that are currently known, including, but not limited to, such properties as the triplet genetic code and specific base pair interactions.

The following examples are provided by way of illustration and not by way of limitation.

### EXAMPLE 1

### Identification and Characterization of a cDNA Encoding a Novel Human Genome-Derived Myosin-Like Gene

In order to identify novel human genes that redispose or contribute to diseases caused by perturbations of the cellular contractile apparatus, we undertook to mine human genomic DNA for exons particularly expressed in human cardiac and/or skeletal muscle, looking in particular for those that are part of genes that have proven refractory to prior identification efforts based upon cloning and/or sequencing of expressed transcripts.

Briefly, bioinformatic algorithms were applied to human genomic sequence data to identify putative exons. Each of the predicted exons was amplified from genomic DNA, typically centering the putative coding sequence within a larger amplicon that included flanking noncoding sequence. These genome-derived single exon probes were arrayed on a solid support and expression of the bioinformatically predicted exons assessed through a series of simultaneous two-color hybridizations to the genome-derived single exon microarrays. The approach and procedures are further described in detail in Penn *et al.,* "Mining the Human Genome using Microarrays of Open Reading Frames," *Nature Genetics* 26:315-318 (2000); commonly owned and copending U.S. patent application no. ("Methods and Apparatus for Predicting, Confirming, and Displaying Functional information Derived from Genomic Sequence," attorney docket no. MDhMORF-1, filed January 29, 2001); and commonly owned and copending U.S. provisional patent application nos. 60/207,456, filed May 26, 2000 ("Human Genome-derived Single Exon Nucleic Acid Probes Useful for Gene Expression Analysis by Microarray"), the disclosures of which are incorporated herein by reference in their entireties.

Using a graphical display particularly designed to facilitate computerized query of the resulting exon-specific expression data, as further described in commonly owned and copending U.S. patent application no. ("Methods and Apparatus for Predicting, Confirming, and Displaying Functional information Derived from Genomic Sequence," attorney docket no. MDhMORF-1, filed January 29, 2001), an exon was identified that is expressed at high levels in human heart but in none of nine other tested tissues or cell types.

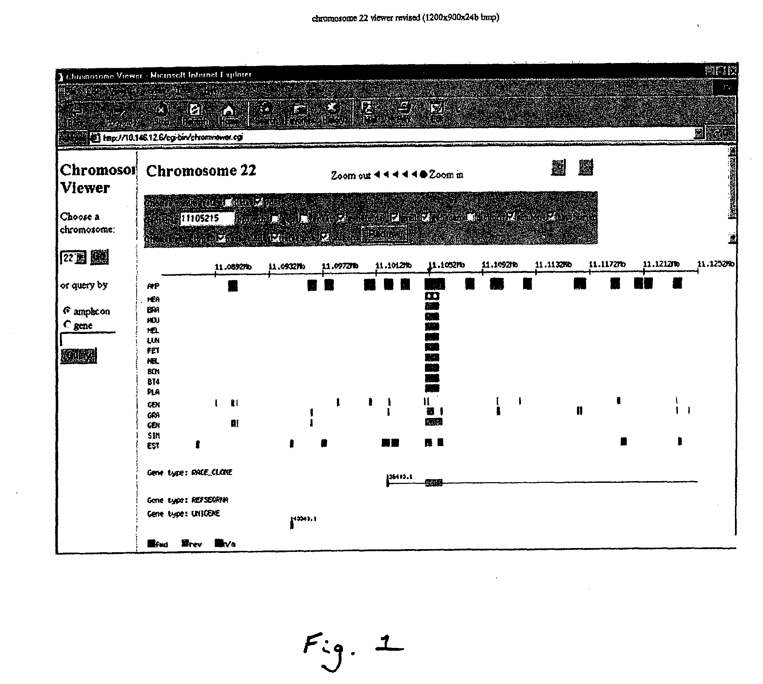

FIG. 1 is a screen shot of the graphical display in which the heart-specific exon is, for purpose of exemplification, centered in the view pane. A black line, demarcated at regular intervals in megabases ("Mb"), represents a continuous stretch of human chromosome 22 ("genome sequence line"). Below the line, in a horizontal field labeled "amp", are blue rectangles that identify regions of chromosome 22 that were, in one or another experiment, amplified from genomic DNA. The blue rectangles are aligned with the genome sequence line to show the starting and ending nucleotides of the amplicons.

Moving downward in the view pane, horizontal fields are presented that show expression measured for various of the amplicons in human heart ("HEA"), brain ("BRA"), adult liver ("ADU"), HeLa cells ("HEL"), lung ("LUN"), fetal liver ("FET"), HBL100 cells ("HBL"), bone marrow ("BON"), BT4 cells ("BT4"), and placenta ("PLA"). As further described in commonly owned and copending U.S. patent application no. ("Methods and Apparatus for Predicting, Confirming, and Displaying Functional information Derived from Genomic Sequence," attorney docket no. MDhMORF-1, filed January 29, 2001), the expression relative to control (here a pool of message from 10 tissues) is shown in shades of red and green, with green indicating expression greater than control.

As can be seen in FIG. 1, the exon centered at about 11.1052 Mb is expressed at levels above control solely (among the tested tissues and cell types) in human heart, meeting our inclusion criteria for further study.

Standard RACE (rapid amplification of cDNA ends) and RT-PCR (reverse transcription polymerase chain reaction) techniques were used to obtain overlapping cDNA clones that collectively span 8,166 nucleotides and appear to contain the entire coding region of the gene. RT-PCR and RACE techniques are described, *inter alia,* in Siebert *et al*. (eds.), Gene Cloning and Analysis by RT-PCR, Eaton Publishing Company/Bio Techniques Books Division, 1998 (ISBN: 1881299147); Schaefer, *Anal. Biochem.* 20:227(2) :255-73 (1995); Ausubel et al. (eds.), Short Protocols in Molecular Biology : A Compendium of Methods from Current Protocols in Molecular Biology, 4^{th} edition (April 1999), John Wiley & Sons (ISBN: 047132938X) and Sambrook *et* al. (eds.), Molecular Cloning: A Laboratory Manual (3^{rd} ed.), Cold Spring Harbor Laboratory Press (2000) (ISBN: 0879695773), the disclosures of which are incorporated herein by reference in their entireties. The cDNA was sequenced on both strands using a MegaBace™ sequencer (Molecular Dynamics, Inc., Sunnyvale, CA, USA).

FIGS. 2A - 2L collectively present the sequence of the cDNA [SEQ ID NO:1] and its translation [SEQ ID NO:2]. For reasons set forth below, we have denominated the novel gene hGDMLP-1.

The hGDMLP-1 cDNA spans 8166 nucleotides and contains an open reading frame from nucleotide 170 through and including nt 7876, predicting a protein of 2568 amino acids (285.3 kD absent post-translational modification). The clone appears full length, with the reading frame opening with a methionine and terminating with a stop codon before a 3' poly-A tail.

Structurally, the N-terminal 600 amino acids, as well as the C-terminal 1100 amino acids (1400-2568) are quite hydrophilic, with fully one third of the amino acids of the first 480 residues strongly acidic or basic.

Motif searches using Pfam (http://pfam.wustl.edu/), SMART (http://smart.embl-heidelberg.de/), and PROSITE pattern and profile databases (http://www.expasy.ch/prosite/), identified several known domains. A myosin head (also denominated myosin motor, large ATPase) domain is encoded by amino acid residues 566 - 1335; a myosin tail domain is located at residues 1422 - 1919. Given the presence of a myosin head and a myosin tail, we have denominated the gene "human genome-derived myosin-like protein-1" ("hGDMLP-1").

In addition to myosin head and myosin tail domains, SMART and Pfam both additionally identified an IQ domain at residues 1336-1358. The IQ domain is a short calmodulin-binding motif containing conserved Ile and Gin residues, suggesting interaction of hGDMLP-1 with calmodulin, which is known to serve as the light chain for myosins I and V.

The sequence of the hGDMLP-1 cDNA was used a BLAST query into the GenBank nr and dbEst databases. The nr database includes all non-redundant GenBank coding sequence translations, sequences derived from the 3-dimensional structures in the Brookhaven Protein Data Bank (PDB), sequences from SwissProt, sequences from the protein information resource (PIR), and sequences from protein research foundation (PRF). The dbEst (database of expressed sequence tags) includes ESTs, short, single pass read cDNA (mRNA) sequences, and cDNA sequences from differential display experiments and RACE experiments.

Although a number of ESTs showed strong sequence similarity, including identity, with short regions of the hGDMLP-1 coding sequence, hGDMLP-1 has not previously been identified as a full length clone. Two full length genes prior-accessioned into GenBank do, however, appear to be closely related.

The first is a murine myosin gene containing a PDZ domain (mysPDZ) (GenBank accession no. BAA93660), further described by Furusawa *et al*., "Isolation of a novel PDZ-containing myosin from hematopoietic supportive bone marrow stromal cell lines," *Biochem*. *Biophys. Res. Commun.* 270(1) :67-75 (2000), the disclosure of which is incorporated herein by reference in its entirety. In the region of greatest similarity ― residues 477 - 2270 of hGDMLP-1 ― hGDMLP-1 and BAA93660 show 40% identity and 60% similarity at the amino acid level, with similarity scored according to the NCBI BLAST standard BLOSUM matrix (Henikoff *et al*., *Proc*. *Natl*. *Acad*. *Sci. USA* 89:10915-10919 (1992); Henikoff *et al*., *Proteins* 17:49-61 (1993)). This region includes both the hGDMLP myosin head domain and myosin tail domain.

hGDMLP-1 is also highly similar across the same region (residues 477 - 2270 of hGDMLP-1) to the human orthologue of the murine PDZ-containing myosin in mouse (accession no. BAA13206), showing 40% identity and 61% similarity to hGDMLP-1 at the amino acid level.

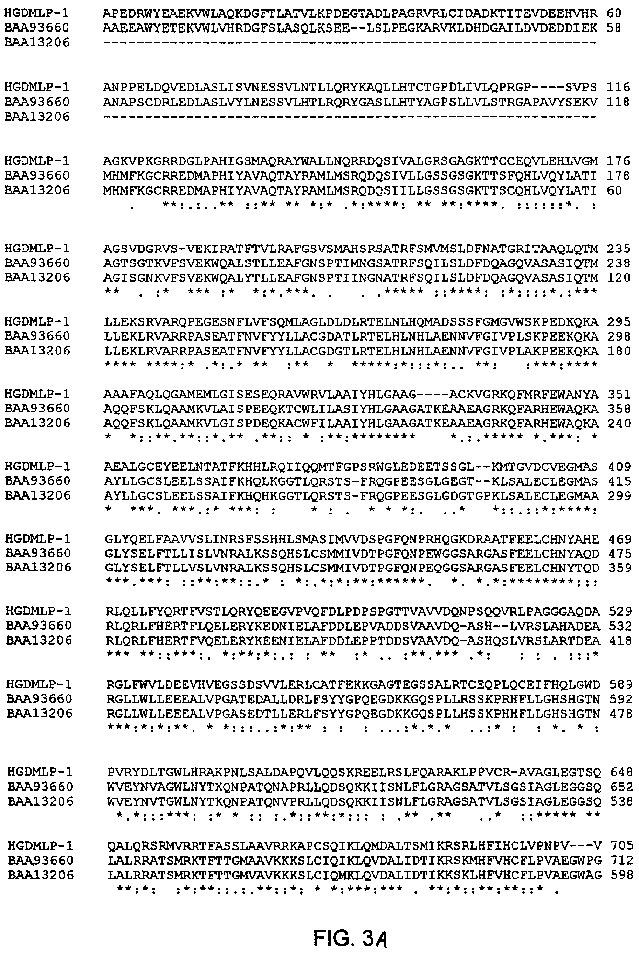

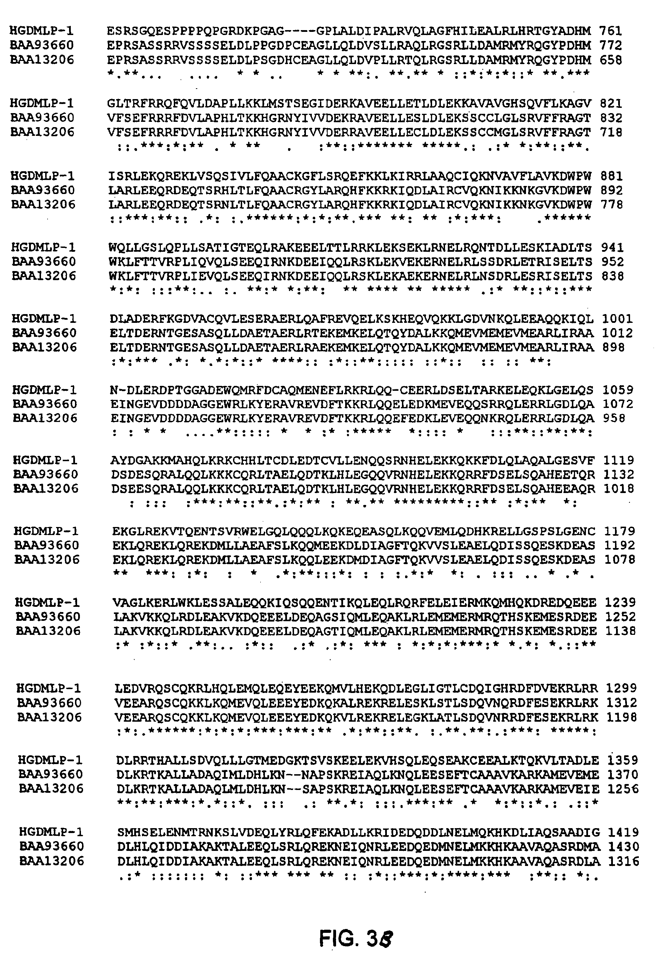

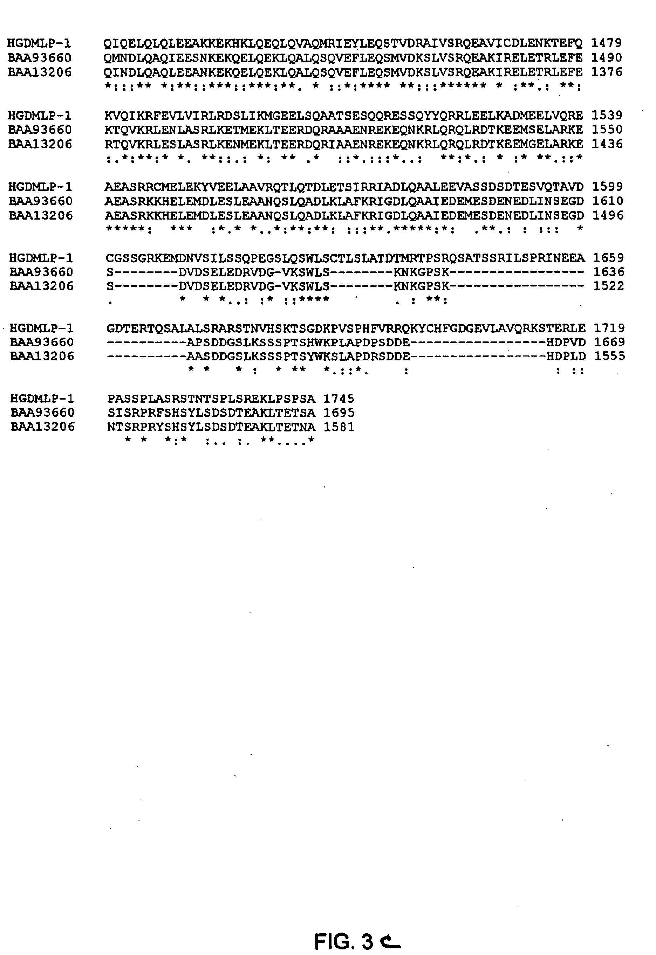

FIGS. 3A - 3C collectively show a multiple sequence alignment of hGDMLP-1 (amino acid residues 505-2249) with BAA93660 (murine PDZ-containing myosin-like gene, residues 341-2035) and BAA13206 (human myosin heavy chain-like gene containing P-loop, residues 1-1581). Gaps are shown within a sequence as one or more dashes (-). In the consensus line underlying the three aligned sequences, "*" indicates residues that are identical or conserved in all sequences in the alignment, ":" indicates conserved substitutions, and "." = indicates semi-conserved substitutions.

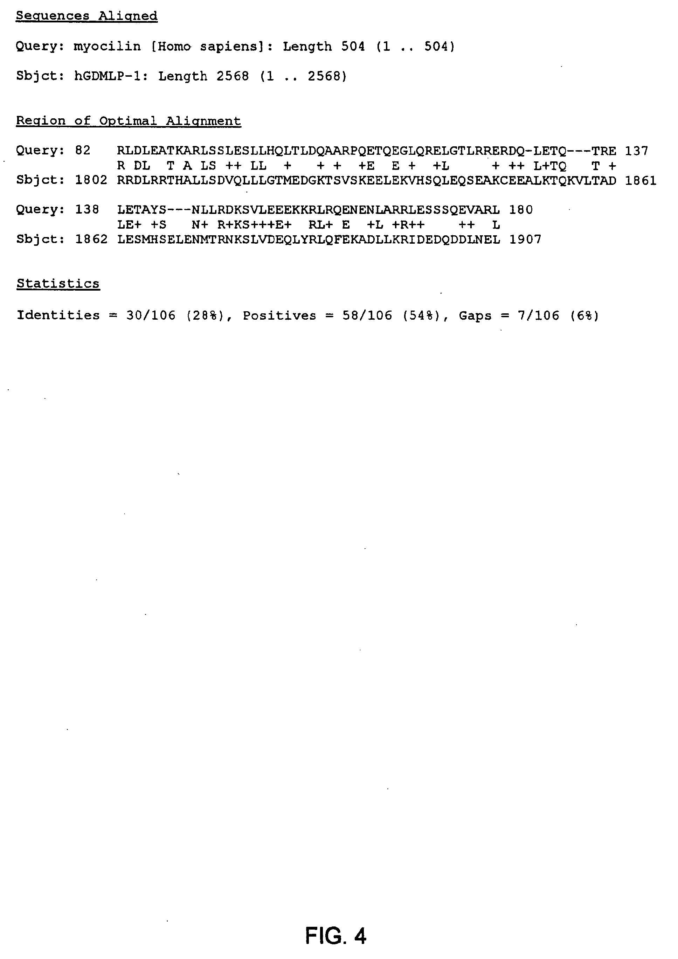

Further directed comparison of hGDMLP-1 with 10 other proteins previously identified to be myosin or myosin-like confirms that hGDMLP-1 is most similar in sequence to mysPDZ. Significant similarity was also detected to human (but not murine) myocilin (myoC). FIG. 4 shows pairwise alignment of hGDMLP-1 to human myocilin (myoC). As shown, in the region of maximal similarity in amino acid sequence, 28% of residues are identical; 54% of residues are similar, with similarity scored according to the BLOSUM 62 matrix.

Both mysPDZ and myoC are cytoskeleton-associated proteins. MyoC is associated with glaucoma, has a secreted form, and is found in association with cilia in photoreceptor cells. Based upon these sequence similarities, hGDMLP-1 is a myosin-like protein that is cytoskeleton-associated.

Separately querying GenBank with sequence drawn from the hydrophilic N-terminus and C-terminus of hGDMLP-1, the N-terminal region (112 - 515) is seen to have some sequence similarity to collagen alpha and other hydrophilic proteins (such as cylicin and neurofilament triplet H protein), whereas the C-terminal end of the protein is unique.

### EXAMPLE 2

### Identification and Characterization of a Genomic Region Encoding hGDMLP-1

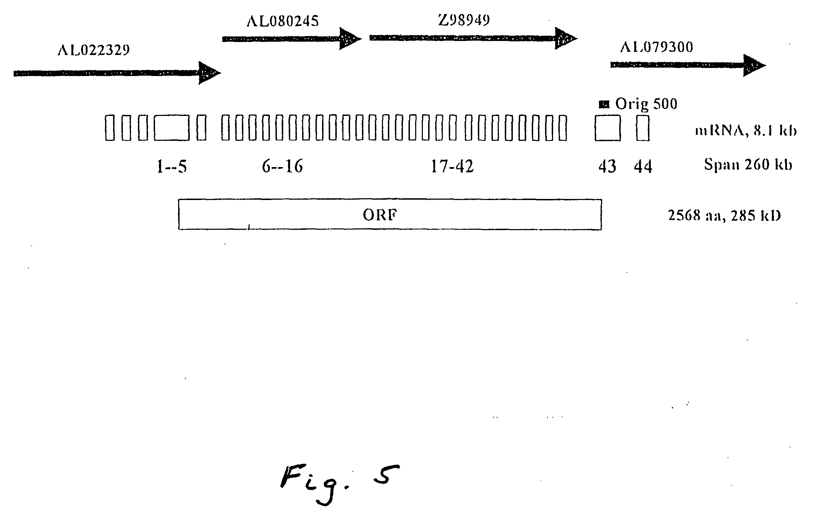

BLAST query of genomic sequence identified four BACs that constitute the minimum set of clones encompassing the cDNA sequence (FIG. 5).

Based upon the known origin of the four BACs (AL022329, AL080245, Z98949 and AL079300) that encompass hGDMLP-1, the gene can be mapped to human chromosome 22q11.2, about 1 Mb distal to the 22q11 low copy repeat region.

Comparison of the cDNA and genomic sequences identified 44 exons, occupying a 288 kb region on chromosome 22q11.2. Exon organization is listed in Table 1.

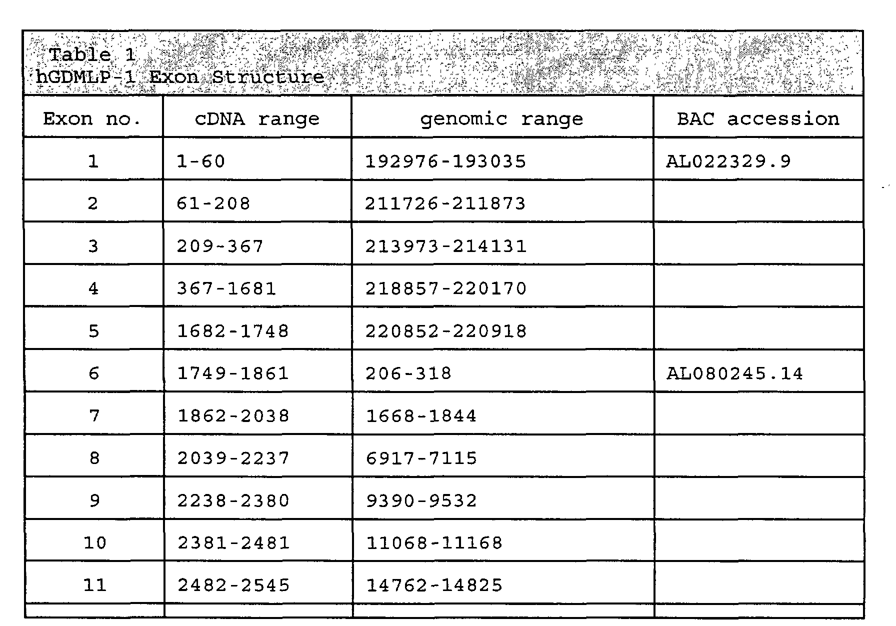

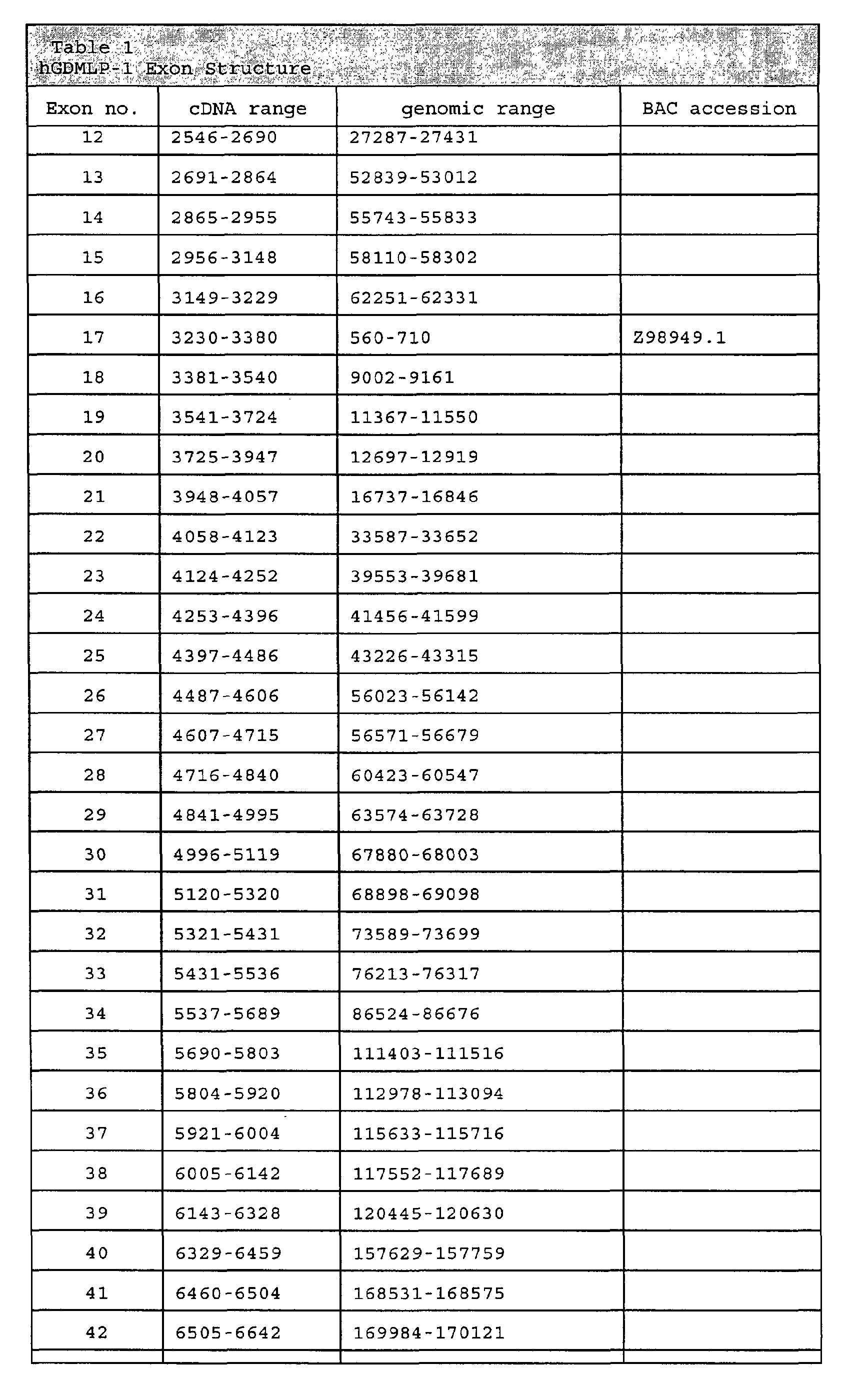

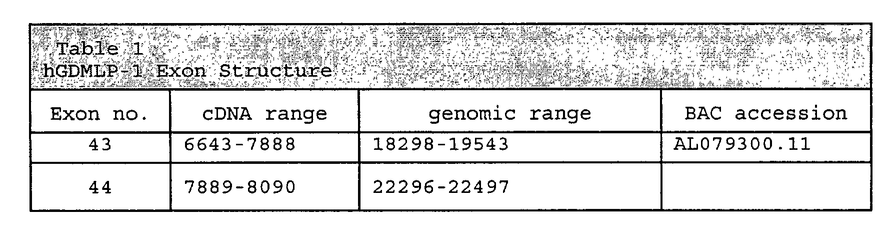

### EXAMPLE 3

### Expression and Transcription Control of hGDMLP-1

Exons 1, 3, 4, 6, 15, 43 and 44 of hGDMLP-1 were separately amplified and the genome-derived single-exon probes arrayed for expression analysis essentially as described in Penn *et al.,* "Mining the Human Genome using Microarrays of Open Reading Frames," *Nature Genetics* 26:315-318 (2000) and commonly owned and copending U.S. patent application no. ("Methods and Apparatus for Predicting, Confirming, and Displaying Functional information Derived from Genomic Sequence," attorney docket no. MDhMORF-1, filed January 29, 2001), the disclosures of which are incorporated herein by reference in their entireties.

Table 2 reports the results of hybridization experiments. "Amplicon" is a unique designation. "Signal" indicates normalized signal intensity. "Ratio" is the ratio of expression relative to control. "ND" indicates "not determined".

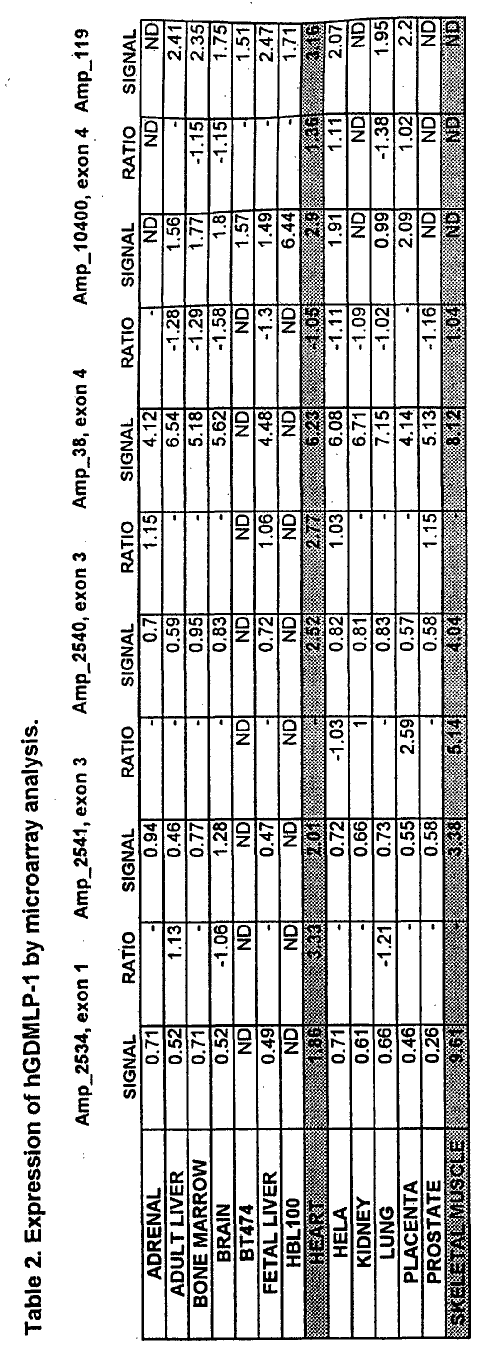

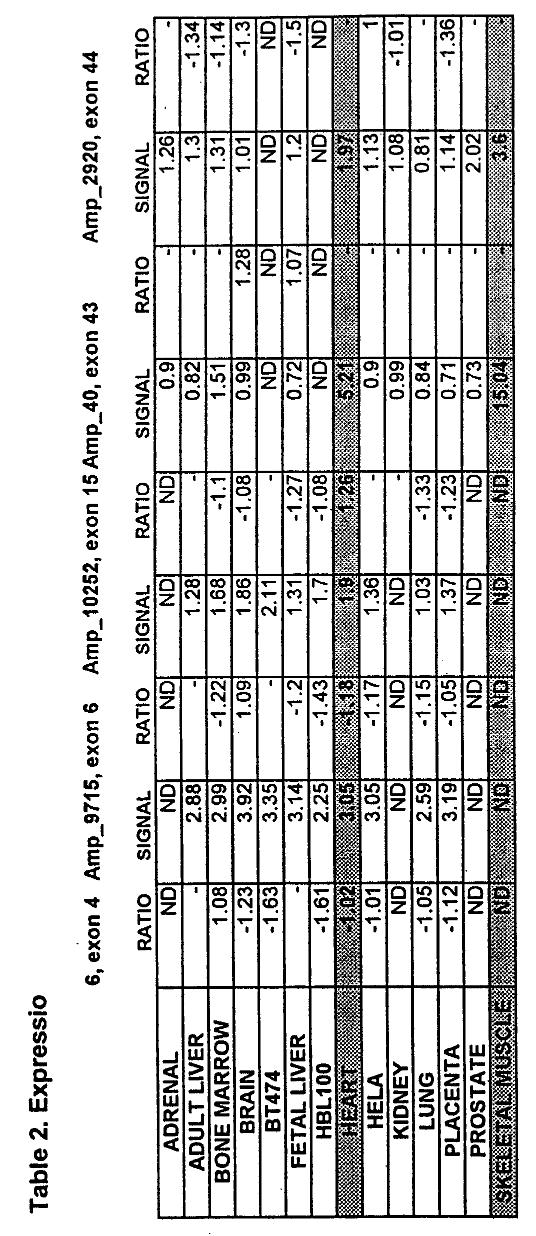

As can be seen in table 2, skeletal muscle and heart are the primary locations of hGDMLP-1 expression.

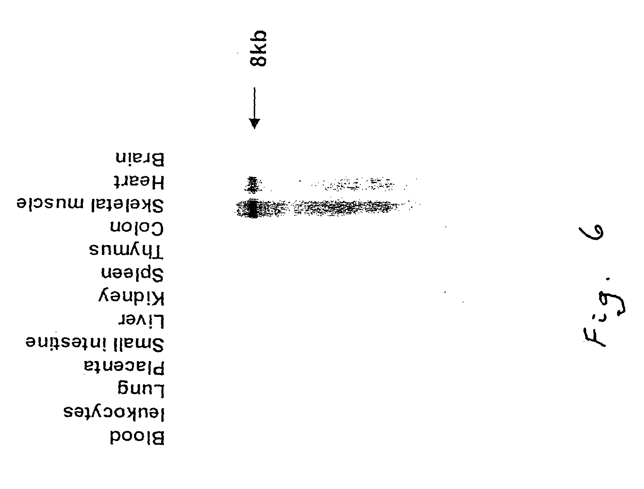

Heart and skeletal muscle-specific expression was further confirmed by northern blot analysis using amplicon 40 (exon 43) as probe. As shown in FIG. 6, of the 12 tissues assayed ― blood, leukocytes, lung, placenta, small intestine, liver, kidney, spleen, thymus, colon, skeletal muscle, heart and brain ― only skeletal muscle and heart demonstrate expression, with both having an 8 kb transcript.

Possession of the genomic sequence permitted search for promoter and other control sequences for the hGDMLP-1 gene.

A putative transcriptional control region, inclusive of promoter and downstream elements, was defined as 2 kb around the transcription start site, itself defined as the first nucleotide of the cDNA clone. The region, drawn from sequence of BAC AL022329, has the following sequence, where nucleotide number 1001 is the transcription start site:

tgattttttttttttttttttgagatggagtctctctgtcacccaggctgga
gtgcagtggcgcgatctcggcttactgcaagctccgcctgtagctgggactac
aggcgcccaccaccacgcccggctaattttgtttttgtatttttagtagagac
ggggtttcactgtgttagccaggatggtctcaatctcctgacctcgtgatatg
cccacctcggcttcccaaagtgccgggattacagacgtgagccaccgtgccag
gcccaaagcactttttatagcccctagtttgctgttgtgtctgtttcctcta
cggcccgcacagcctgggtttgaattctggctctccatggccttgggcaagtt
ggttaactttttgtgtctcaatttccccacttgtaaagtggggctggggaaaa
accctacctcatagggaacattaagtgagttcatgaaagccctcggtgtgtgg
catgcgataaccactacctaagcataaggtatgtttacatgcactggttgttt
accacgacgcaccagcattcattcaagttattttcttccagagtctgcctgga
atgtagaaccggccagctctcctcctcaaacagttcctgcaacctctggttat
ctgtgtgggcagaggcgggagtgtgggtgtgtggccctgccctgggacaggga
tgacgagagagcatgtgcttgtgtccagagctgtccccttccctgctcagggg
ccaccaaatatgtccatggtctctgctgcctggtcaacggccaccccgggctg
ccgtctcctggccagacctggagctcacaagctaattttagaaatgactggtc
gactcagctgctgctatcacattggcccagaaggagggtgggggaggacggg

tcggggaggggcagtaaggggaagggatgtgtgtcggtgtgtgtggagagGGT
GCTCCTTCGCTCCTGCTTTCCCGGCTCGGTGGGGTCCCTTCGCGGcacccgg
ggagacgtcttagacagtcgtggttcctgtgatctgtgtcctctttgcagaag
gtactattgggctgggttttagacgggcaccttgcgagcggatcggcgagttt
taattttccggaatgcgctgctgggggctgcggtggcaggcctccggtctttt
ggtctattttaggcctttctggtgttgagagcttccgaggcctgattcctttg
cctgttgcccgtgctgctcactttgaaaacaagagtgtttcggtggaaggatt
tgagaacaaaatgctggggcaaatggacattcgtaagaatcactctcctcagg
ccccagctgttcgctaataatttttaaaaatgtgtttagaatcatgagttaat
ctccactgtgaatctttgcccgcagacgtcagggtttatatttttcctcaact
accagtaatttctgcttcactggtgttcagttatggtcggggaagagccaggc
ttcccctttcgtgttaacaacgctgttgagtcgaaatgttaattttaatgttc
tttttaaactgcaaattaagcatgtatgagtgttgggggaagggcaatggaa
gttcatggctgaaggtttcaatttcatgccaccttccttgagctctgtctgag
ggaaggtgtcttatctggcaccccggatagcaggcatttctctgaatgaagaa
gattcacaggtcccagtggccccatagtggggcagaagggggctatgctttga
catggagctctctcccaggagaggagaagacctcaccagctacaggcctctgg
gctgctgccttcctgcagcttttccatggaggaggccaggagtggggaggccc
ggaatggggagcccagtcggctcccagtcggctccaagctgtccagtctggcc
tcaacccgcaagtgtggggatctgggcttctatcctgtagggcctggaatacg
acgaggctgttactttcactttgtggctgtccaaaaaga [SEQ ID NO:3]

Using PROSCAN,
(http://bimas.dcrt.nih.gov/molbio/proscan/), no significant promoter was identified in the putative promoter region. However, transcription factor binding sites were identified using a web site at http://motif.genome.ad.jp/, including a SRY (sex-determining region Y gene product) binding sites (134..140 bp, with numbering according to SEQ ID NO:3), and a couple of MZF1 (myeloid zinc finger 1, a negative regulator of CD34 and c-myb, and responsive to retinoic acid) binding sites (1843..1850 bp and 403-395 bp).

### EXAMPLE 3

### hGDMLP-1 Disease Associations

Diseases that map to the hGDMLP-1 chromosomal region are shown in Table 3.

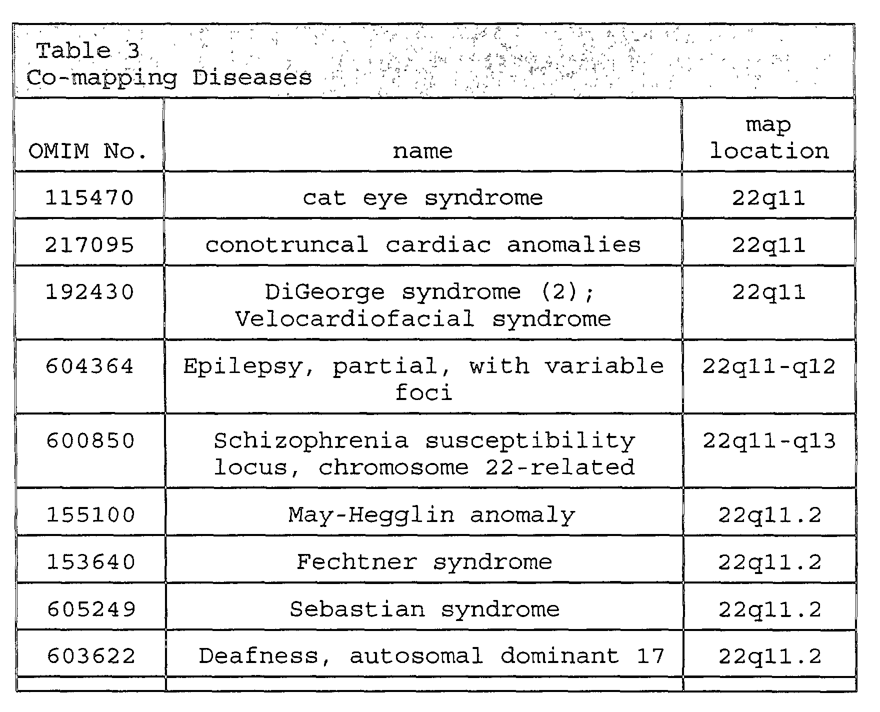

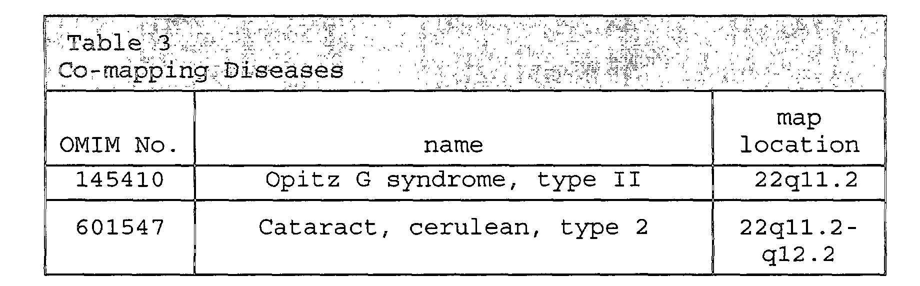

A number of these diseases have physiologic characteristics consistent with alteration of a myosin-like gene, especially a myosin-like gene particularly expressed in human heart.

Thus, the iris alterations that give cat eye syndrome (CES) its name are frequently associated with heart malformations. A small supernumerary chromosome (smaller than chromosome 21) is present, frequently has 2 centromeres, is bisatellited, and represents an inv dup(22)(q11). No gene candidates have been identified as yet.

Individuals with 22q11 deletion syndrome (del 22q11) have a range of findings, including congenital heart disease (74% of patients), particularly conotruncal malformations (tetralogy of Fallot, interrupted aortic arch, and truncus arteriosus); palatal abnormalities (69%), particularly velopharyngeal incompetence (VPI), submucosal cleft palate, and cleft palate; characteristic facial features (present in the majority of individuals); and learning difficulties (70 - 90%).

DiGeorge syndrome (DGS), most cases of which result from a deletion of chromosome 22q11.2, is characterized by hypocalcemia arising from parathyroid hypoplasia, thymic hypoplasia, and outflow tract defects of the heart. This syndrome has previously been considered to be a contiguous gene deletion, but one or a few genes in the identified locus may play a major role in the pathogenesis of the syndrome's characteristic phenotypic features. Several genes are lost in the characteristic deletion, but definitive identification of the causative genes is lacking.

Several disorders have been tentatively linked to another myosin-like gene that maps to 22q11, MYH9, although some of these associations are inferential and may be due instead to mutations in hGDMLP-1.

For example, the autosomal dominant giant-platelet disorders May-Hegglin anomaly, Fechtner syndrome, and Sebastian syndrome share the triad of thrombocytopenia, large platelets, and characteristic leukocyte inclusions. All three disorders may be allelic since May-Hegglin anomaly and Fechtner syndrome map to an overlapping region of 480 kb on chromosome 22. MYH9 was recently identified as a possible candidate gene, since MYH9 is expressed in platelets and is upregulated during granulocyte differentiation, but association of the disease with MYH9 is inferential. Kelley *et al., Nature Genetics* 26:106-108 (2000).

Other disorders that map to 22q11 may result from defects of hGDMLP-1, although primary disease manifestions are not in cardiac or skeletal muscle.

For example, Xiong *et al., Am. J. Hum. Genet.* 65:1698-1710 (1999), mapped the disease locus for a familial partial epilepsy syndrome with variable foci to an interval on 22q11-q12. The syndrome is characterized by mostly nocturnal seizures arising from frontal, temporal, and occasionally occipital epileptic foci. The syndrome is inherited as an autosomal dominant trait with incomplete penetrance.

Several studies have suggested that there may be a susceptibility locus for schizophrenia located on chromosome 22. Gill *et al*., *Am. J. Med. Genet.* 67:40-5 (1996). Additionally, Pulver et al. (1994) reported a higher incidence of schizophrenia among patients with velocardiofacial syndrome, which is localized to 22q11. Candidate genes have not yet been identified.

Opitz G syndrome, Opitz *et al*., *Birth Defects Orig. Art. Ser.* 2: 95-101 (1969), is characterized by hypertelorism or telecanthus; laryngotracheoesophageal cleft; clefts of lip, palate, and uvula; swallowing difficulty and hoarse cry; genitourinary defects, especially hypospadias in males and splayed labia majora in females; mental retardation; and congenital heart defects. Opitz Syndrome may also be characterized by additional abnormalities, including partial or complete closure of the anal opening (imperforate anus); hypoplasia or agenesis of the corpus callosum; renal abnormalities; heart defects; or mental retardation. Although Opitz G syndrome has been mapped to 22q11.2, no specific genes have been associated with the disease. *See, e.g., Robin et al*., *Am*. *J*. *Med*. *Genet.* 62: 305-317 (1996).

Congenital cataracts are responsible for between 10 and 30 % of all cases of blindness in children. One of these congenital forms is cerulean cataract which has been mapped to 22q11.2-22q12.2. The beta-crystallin genes have been identified as possible candidate genes, but no definitive association has been shown.

All patents, patent publications, and other published references mentioned herein are hereby incorporated by reference in their entireties as if each had been individually and specifically incorporated by reference herein. By their citation of various references in this document, applicants do not admit that any particular reference is "prior art" to their invention.

While specific examples have been provided, the above description is illustrative and not restrictive. Any one or more of the features of the previously described embodiments can be combined in any manner with one or more features of any other embodiments in the present invention. Furthermore, many variations of the invention will become apparent to those skilled in the art upon review of the specification. The scope of the invention should, therefore, be determined not with reference to the above description, but instead should be determined with reference to the appended claims along with their full scope of equivalents.

## Claims

1. An isolated nucleic acid molecule selected from the group consisting of: (a) an isolated nucleic acid molecule that encodes the amino acid sequence of SEQ ID NO:2; (b) an isolated nucleic acid molecule that encodes a fragment of at least 10 amino acids of SEQ ID NO:2; (c) an isolated nucleic acid molecule that encodes an amino acid sequence having at least about 65% amino acid sequence identity to SEQ ID NO:2; (d) an isolated nucleic acid which hybridizes under high stringency conditions to a nucleic acid molecule comprising SEQ ID NO:1; and (e) an isolated nucleic acid complementary in sequence to the nucleic acids of (a) - (d).

2. The isolated nucleic acid molecule of claim 1, wherein said nucleic acid molecule comprises sequence of SEQ ID NO:1 or its complement.

3. The isolated nucleic acid molecule of claim 2, wherein said nucleic acid molecule comprises nucleotides 170 - 7876 of SEQ ID NO:1 or the complement thereof.

4. The isolated nucleic acid molecule of any one of claims 1 - 3, wherein said nucleic acid molecule is operably linked to one or more expression control elements.

5. A replicable vector comprising an isolated nucleic acid molecule of any one of claims 1 - 4.

6. The isolated nucleic acid molecule of any of claims 1 - 3, attached to a support.

7. A host cell transformed to contain the nucleic acid molecule of any one of claims 1 - 5, or the progeny thereof.

8. A method for producing a polypeptide, the method comprising: culturing the host cell of claim 7 under conditions in which the protein encoded by said nucleic acid molecule is expressed.

9. An isolated polypeptide produced by the method of claim 8.

10. An isolated polypeptide selected from the group consisting of: (a) an isolated polypeptide comprising the amino acid sequence of SEQ ID NO:2; (b) an isolated polypeptide comprising a fragment of at least 10 amino acids of SEQ ID NO:2; (c) an isolated polypeptide according to (a) or (b) in which at least 95% of deviations from the sequence of (a) or (b) are conservative substitutions; and (d) an isolated polypeptide having at least 65% amino acid sequence identity to the isolated polypeptides of (a) or (b).

11. An isolated antibody or antigen-binding fragment or derivative thereof the binding of which can be competitively inhibited by a polypeptide of any one of claims 9 or 10.

12. A method of modulating at least one activity of a polypeptide according to claim 10, the method comprising:
administering an effective amount of an agent which modulates at least one activity of a polypeptide according to claim 10.

13. A method of diagnosing a disease caused by mutation in hGDMLP-1, comprising:
detecting said mutation in a sample of nucleic acids that derives from a subject suspected to have said disease.

14. A method of diagnosing or monitoring a disease caused by altered expression of hGDMLP-1, comprising:
determining the level of expression of hGDMLP-1 in a sample of nucleic acids or proteins that derives from a subject suspected to have said disease, alterations from a normal level of expression providing diagnostic and/or monitoring information.

15. A pharmaceutical composition comprising the nucleic acid of any one of claims 1 - 5 and a pharmaceutically acceptable excipient.

16. A pharmaceutical composition comprising the polypeptide of claim 9 or claim 10 and a pharmaceutically acceptable excipient.

17. A pharmaceutical composition comprising the antibody or antigen-binding fragment or derivative thereof of claim 11 and a pharmaceutically acceptable excipient.

18. A diagnostic composition comprising the nucleic acid of any one of claims 1 - 5, said nucleic acid being detectably labeled.

19. A diagnostic composition comprising the polypeptide of claim 9 or claim 10, said polypeptide being detectably labeled.

20. A diagnostic composition comprising the antibody or antigen-binding fragment or derivative thereof of claim 11.

21. The diagnostic composition of claim 20, wherein said antibody or antigen-binding fragment or derivative thereof is detectably labeled.

22. A microarray wherein at least one probe of said array is a nucleic acid according to any one of claims 1 - 5.

23. A method for detecting a target nucleic acid in a sample, said target being a nucleic acid of any one of claims 1 - 5, the method comprising:
a) hybridizing the sample with a probe comprising at least 30 contiguous nucleotides of a sequence complementary to said target nucleic acid in said sample under hybridization conditions sufficient to permit detectable binding of said probe to said target, and
b) detecting the presence or absence, and optionally the amount, of said binding.

24. A fusion protein, said fusion protein comprising a polypeptide of claim 10 fused to a heterologous amino acid sequence.

25. The fusion protein of claim 24, wherein said heterologous amino acid sequence is a detectable moiety.

26. A method of screening for agents that modulate the expression of hGDMLP-1, the method comprising:
contacting a cell or tissue sample believed to express hGDMLP-1 with a chemical or biological agent, and then
comparing the amount of hGDMLP-1 expression with that of a control.


<110> AEOMICA INC.

<120> MYOSIN-LIKE GENE EXPRESSED IN HUMAN HEART AND MUSCLE

<130> PB 0105-EP

<140> EP 01112637.2-2105
<141> 2001-05-24

<150> US 60/207,456
<151> 2000-05-26

<150> US 60/236,359
<151> 2000-09-27

<150> PCT/US01/00666
<151> 2001-01-30

<150> PCT/US01/00667
<151> 2001-01-30

<150> PCT/US01/00664
<151> 2001-01-30

<150> PCT/US01/00669
<151> 2001-01-30

<150> PCT/US01/00665
<151> 2001-01-30

<150> PCT/US01/00668
<151> 2001-01-30

<150> PCT/US01/00663
<151> 2001-01-30

<150> PCT/US01/00662
<151> 2001-01-30

<150> PCT/US01/00661
<151> 2001-01-30

<150> PCT/US01/00670
<151> 2001-01-30

<150> US 60/234,687
<151> 2000-09-21

<150> US 60/266,860
<151> 2001-02-05

<160> 3

<170> Aeomica Sequence Listing Engine

<210> 1
<211> 8117
<212> DNA
<213> Homo sapiens

<220>

<221> CDS
<222> 170-7876

<400> 1
cacccgggga gacgtcttag acagtcgtgg ttcctgtgat ctgtgtcctc tttgcagaag 60
ttctgtgtcc atctcatgtg ctgcgtgtgt ctgtaaagcc tcattccgtg ctgtctggca 120
ggaagctcca tctcatctca tcatctcacg gccctggcac tgcctcagca tggccatctc 180
atcacgcctc gccctgtggg agcagaagat tcgggaagag gacaagagcc ctccaccatc 240
ctcgccccct cctcttttct ctgtcatccc agggggcttc attaagcaac tggtccgggg 300
gactgaaaaa gaggccaagg aagcgagaca gaggaagcag ttagctgtcg cctctccaga 360
acgagagatc ccagaaattt ccatcagcca acccaacagc aagtccagca gtggcaccag 420
atctggaagc cagcagatct ctcaggacga ccagtcaagc tctcctggga gctcagacat 480
tctgggcaag gagagcgagg ggtcccgcag ccccgaccct gagcagatga caagcatcaa 540
tggtgagaag gcccaggagc tgggctccag tgcgacacca accaaaaaga ctgtcccctt 600
caagaggggc gtgaggaggg gtgatgtgtt gttgatggtg gccaagctgg acccggactc 660
agccaagcca gagaagactc atccccatga cgccccccct tgcaagacct ctccccccgc 720
cacagatact ggaaaggaaa agaaagggga gacctctagg actccttgtg gctcccaggc 780
cagcaccgag atcttggccc cgaaagctga gaagacccgg actgggggtc ttggggaccc 840
aggccaagga actgtggcac tgaaaaaagg cgaggagggt caaagcatag tggggaaggg 900
gcttgggacc cccaagacca cagagctgaa agaggctgag ccccagggca aagacaggca 960
ggggaccagg ccccaagccc aagggcccgg cgagggggtg cgaccaggga aagcagagaa 1020
ggagggagca gagcccacaa acacggtgga aaaggggaat gtctctaagg acgtagggag 1080
tgaagggaag cacgtaaggc cccaaatccc tgggagaaag tggggaggtt tcctgggaag 1140
aaggagtaag tgggacggtc cccagaataa gaaggacaaa gaaggggtgc tcttaagtaa 1200
ggcagagaag acaggtgagc ctcagaccca gatggagaag acaagccaag tgcagggcga 1260
gttgggggac gatctgagaa tgggggagaa agcaggtgag cttcggagca cgactgggaa 1320
ggcaggtgag tcctgggata agaaggaaaa gatggggcaa ccccagggta agtccgggaa 1380
cgcaggtgaa gctcggagtc agacagagaa gggctgtgaa gccccaaagg aggtgagcac 1440
aatggtggag tcgccagcag ctcctgggaa gggaggctgg ccaggaagcc gtgggcagga 1500
agcagaggag ccctgctcaa gagcaggtga tggggctggt gccctggaga cagagctgga 1560
aggacccagc cagcctgctc tggagaagga tgcagaaagg cctcggatac ggaaggagaa 1620
ccaagacggg ccagccccgc aggaggaggg caaaggaggc cagagcagag actcagacca 1680
ggctcctgag gacagatggt atgaggcaga gaaagtctgg ctggctcaga aggatggatt 1740
tactcttgct acggtgctaa agccagatga gggaacagca gacctgccag caggaagggt 1800
gagactttgc attgatgctg acaaaaccat cactgaggtg gatgaggagc atgtccatcg 1860
ggccaaccct cctgagctgg accaggtcga ggacctggcc tctctcatca gtgtcaacga 1920
atccagtgtc ctgaacacgc ttctgcagcg ctacaaagct cagctgctgc acacctgcac 1980
agggcctgat ctgattgtcc tccagccccg ggggccctcg gtgccttctg cagggaaggt 2040
gcccaagggc cgccgggatg gcctgcctgc ccacattggc tccatggcac agcgggcata 2100
ctgggcgctg ctgaaccagc ggagagacca gagcattgtg gccctgggcc ggagtggcgc 2160
tgggaagacc acctgctgtg agcaggtcct ggaacacctg gtggggatgg caggcagtgt 2220
ggatggcagg gtctcagtgg agaagatccg agccaccttc actgtcctcc gggccttcgg 2280
ctctgtgtcc atggcccaca gccgcagtgc cacccggttc tccatggtga tgtcgctgga 2340
cttcaacgct acaggccgca tcacagctgc tcagctccag acaatgcttt tggagaagag 2400
ccgcgtggca cggcagccgg aaggggaaag taacttcctg gttttctccc agatgctggc 2460
tggattggac ttggatctca ggacggagct gaacctgcac cagatggcag atagcagctc 2520
ctttggcatg ggcgtgtggt ccaagcctga agataaacag aaggcggcag ctgcctttgc 2580
ccagctccag ggtgccatgg agatgctggg catctcagag agcgagcagc gggctgtttg 2640
gcgggtcctg gcagccatct accacctggg cgcggcgggg gcctgcaaag tgggtcggaa 2700
gcagttcatg aggtttgagt gggcaaacta cgcagctgag gccctgggct gcgagtatga 2760
ggagctgaac acggccacct tcaagcacca ccttcgacag atcatccagc aaatgacgtt 2820
tgggccaagc cgatggggcc tcgaggatga ggaaaccagc tcagggctca agatgacagg 2880
agtggactgt gtggagggga tggcctcggg cctgtaccag gaactctttg cggctgtggt 2940
ctcactcatc aacagatcct tttcctccca ccatctctcc atggcctcca tcatggtggt 3000
ggactctcca ggcttccaga acccccggca ccagggcaag gaccgggcgg ccacctttga 3060
ggagctgtgc cacaactacg cccatgagcg cctgcagctg ctgttctacc agcggacctt 3120
tgtctccacg ctacagcgat atcaagagga aggtgttcct gtgcagtttg acctcccgga 3180
cccctcccca gggaccaccg tggctgttgt ggatcaaaat ccctctcagc aggtccgctt 3240
accagctgga ggaggtgccc aggatgccag aggccttttc tgggtcttag atgaggaagt 3300
ccatgtagag ggctccagtg acagtgtggt gctcgagcgt ctgtgtgcta ctttcgagaa 3360
gaaaggagct gggactgaag ggtcctctgc cctgcggacc tgtgagcagc ccctccagtg 3420

tgagattttc caccagttgg gatgggaccc tgtgcggtac gacctcacgg gctggctcca 3480
cagagccaag cccaacctct cggccctgga tgcaccccag gtcctgcaac agtcaaaaag 3540
agaggagctg cggagtctat tccaggcccg ggccaagctg cctcctgtgt gccgggctgt 3600
ggcaggcctg gagggcacct cccagcaggc cctgcagagg agccgcatgg tgaggaggac 3660
ctttgccagc agccttgccg cggtgaggag gaaagccccg tgctcccaga tcaagctgca 3720
gatggatgcg ctgaccagca tgatcaaaag gtcccggctg cactttatcc actgcctggt 3780
accaaaccct gtggtggaaa gcaggagtgg gcaggaatct ccaccaccac cgcagcctgg 3840
tagagacaag cctggggcag gtggacctct ggccctggat atcccagcac tgagggtcca 3900
gcttgctggg ttccacatcc tggaggctct gcgtctgcat aggacaggct atgctgacca 3960
catggggctc actcgcttcc gccggcaatt ccaggtgctg gacgctccac tcctgaagaa 4020
gctcatgtcg acctccgagg gaatagatga aaggaaggcc gtggaggagc tcctggagac 4080
cctggatctg gaaaagaagg cggtggctgt ggggcacagc caagtttttc tcaaggcagg 4140
tgtgatctcc aggcttgaga agcagcgaga gaagctggta tctcagagca tcgttctctt 4200
ccaggcggct tgcaagggct ttctgtctcg ccaggaattc aagaagctga agattcgccg 4260
actggctgca cagtgcatcc agaagaatgt ggctgtgttc ctcgcagtca aggactggcc 4320
atggtggcag ctgcttggtt ccctccagcc tctacttagt gccaccattg gaactgagca 4380
gctccgagcc aaggaggagg agcttacaac gctaagacgg aagctagaaa aatcagagaa 4440
gttgcggaat gaactccggc agaacacaga tctgctagaa agcaagattg ctgacttgac 4500
ctctgacctt gccgatgagc gcttcaaagg tgatgtggcc tgccaggtgc tggagagtga 4560
gcgggcagag cggctacagg ccttccggga ggtccaggag ctcaagagca agcatgaaca 4620
agtccagaaa aaactgggag atgtgaataa acagttggaa gaagcccagc agaaaattca 4680
gttgaatgac ttggaaaggg atcccactgg aggagcagac gagtggcaga tgcgcttcga 4740
ctgtgctcag atggagaacg agttcctcag aaagcgtctg cagcaatgcg aggagaggct 4800
ggactcggag ctgacagcca ggaaagagct ggagcaaaag cttggggagt tgcaaagtgc 4860
ttatgacggg gccaagaaga tggctcacca actgaagagg aagtgccacc atcttacctg 4920
tgaccttgag gatacctgcg tcctgctaga gaaccaacaa agtcgaaacc atgagctgga 4980
gaagaagcag aagaagtttg acctgcagct ggcccaggcc ctaggtgagt cagtgtttga 5040
gaagggtctc cgtgagaaag tgacccagga gaacaccagt gtccggtggg agctaggcca 5100
gcttcagcag cagctgaagc aaaaggagca ggaagcctca cagctgaagc agcaggtgga 5160
gatgctacag gaccataaac gggagctgct ggggtcaccc tctctggggg aaaattgcgt 5220
tgctggcttg aaggagaggc tctggaagtt ggaatccagc gcccttgagc aacagaaaat 5280
ccagagccag caggaaaaca ccatcaagca gctggagcag ctccgccagc ggtttgagct 5340
ggagatcgag cggatgaagc agatgcacca gaaggaccgt gaggaccagg aggaggaact 5400
ggaggatgtc cgtcagtcct gccagaagcg gcttcatcag ctggaaatgc agctggagca 5460
agagtatgaa gagaagcaga tggtcctcca tgagaagcaa gatttggaag gcttgatcgg 5520
aaccctctgt gaccagattg gccatcggga ctttgatgtg gagaagcgac ttcggagaga 5580
cctcaggagg acacatgcac tgttgtcaga cgtgcagctc cttctgggca ccatggagga 5640
tggcaagaca tcagtcagca aggaggagct ggagaaagtg cacagccagc tggagcagag 5700
tgaagccaag tgtgaggagg ccttgaagac gcagaaggtg ctcacagcgg acctggagag 5760
catgcacagc gagctggaga acatgacgcg gaacaagagc ctggtggatg agcagctgta 5820
caggctgcag tttgagaagg cggacctcct gaagcgcatc gatgaggacc aggatgacct 5880
gaatgagctg atgcagaagc acaaggacct cattgctcag tctgctgctg acattggcca 5940
gatccaagaa ctgcagctgc agctggagga agccaagaag gagaagcaca agctacaaga 6000
acaactgcag gtggctcaga tgcgcatcga gtacctggaa cagtccaccg tggatcgagc 6060
catcgtcagc aggcaggagg cggtcatctg tgacctagag aacaagacag agttccagaa 6120
ggtgcagatt aagagatttg aggtcctggt gatccggctt cgggacagcc tgatcaagat 6180
gggggaggag ctttcacagg cggccacctc cgagtcccag cagcgggaga gcagccagta 6240
ctaccagcgg cgcctggaag agctgaaggc cgacatggaa gagctggtgc agcgggaggc 6300
agaggccagc cggcggtgca tggagctgga gaagtacgtg gaggaacttg cagcagtgag 6360
gcaaaccctc cagacagacc tggagacatc cattcggcgg attgccgacc tgcaggctgc 6420
cttggaagaa gtggcatcca gtgacagtga tactgagagt gtccagacgg cagtggattg 6480
tggcagcagc ggccgaaaag agatggataa cgtctccatc ctcagctccc agccagaggg 6540
cagcctgcag tcctggttga gctgtactct gtccctggcc acagatacta tgaggactcc 6600
ttctcgacag tcagccacca gcagccgcat cctcagcccc aggataaacg aagaggctgg 6660
ggacactgag aggacccagt cggcattggc actgagcaga gcccggtcca ccaatgtcca 6720
cagcaagacc tcaggagaca agcctgtttc tccccacttt gtccgccggc aaaagtactg 6780
tcatttgggg gacggcgaag tgcttgccgt ccagagaaag tccacagaga gattagaacc 6840
tgcttcctct cccctggctt ctcggagtac aaatacatcc ccgctgtcga gggaaaagct 6900
gcccagtcct tcagcggccc tctcggagtt cgtggaaggg ctccggagga agagagccca 6960
gagaggccag gggtccacgc tgggcctaga ggactggccc actctcccca tttaccagac 7020
gactggggcc tccacactaa ggaggggcag ggctggcagt gacgagggaa acctctcgct 7080

gagggttggg gcaaagtcac ccctggaaat cgaaggggcc gctggtggtc tcttgaggtc 7140
caccagcctc aaatgcatct cttcagacgg tgttgggggc acaaccctac tccccgaaaa 7200
gtcgaaaacc caattcagtt cctgcgagtc cctcttagaa tccagaccga gcatggggag 7260
aaaactgagc tctccgacca cacccaggga catgctgttg tcgcccacac tgcgtcctcg 7320
gaggcggtgt ctggagtcct ctgtggacga tgcgggctgt ccagaccttg gaaaggagcc 7380
gcttgttttc cagaaccgcc agtttgccca cctgatggag gaacctctag gcagtgaccc 7440
attcagctgg aaactcccaa gcctcgacta cgaacgcaag accaaagtgg acttcgatga 7500
cttcctccca gctatccgga agccccagac acctacctcc ttggctggat cagccaaagg 7560
tgggcaagac ggttcacagc gttcaagcat ccactttgaa acggaagagg ctaaccgttc 7620
ctttctctcg gggatcaaga ccattttgaa gaagagcccg gagcccaagg aggatcccgc 7680
tcacctgtct gactcgtcct catcctccgg ctccatcgtg tccttcaaaa gtgctgacag 7740
catcaaaagt cgaccaggaa tcccacgact tgcgggtgac ggtggcgagc gaacgtcccc 7800
cgagcggaga gagccaggga cggggaggaa agacgacgat gttgcgagca taatgaagaa 7860
atacctccag aagtaggaac cagttcagcc tccttgaagc tgcccttgaa gacttcccga 7920
ctctacaata acttggagac agagagactg gccaggcctc cccggtggcc agagccagcc 7980
agcatggcca ccctcaagag gcgagatgag cccacagagg catatcctgc ggggatgctg 8040
ggctcccagt gtggttggcc tgaacaaaat aaagtgttga ctcctgggca aaaaaaaaaa 8100
aaaaaaaaaa aaaaaaa 8117

<210> 2
<211> 2568
<212> PRT
<213> Homo sapiens

<400> 2

<210> 3
<211> 1000
<212> DNA
<213> Homo sapiens

<400> 3
tgattttttt tttttttttt gagatggagt ctctctctgt cacccaggct ggagtgcagt 60
ggcgcgatct cggcttactg caagctccgc ctgtagctgg gactacaggc gcccaccacc 120
acgcccggct aattttgttt ttgtattttt agtagagacg gggtttcact gtgttagcca 180
ggatggtctc aatctcctga cctcgtgata tgcccacctc ggcttcccaa agtgccggga 240
ttacagacgt gagccaccgt gccaggccca aagcactttt ttatagcccc tagtttgctg 300
ttgtgtctgt ttcctctacg gcccgcacag cctgggtttg aattctggct ctccatggcc 360
ttgggcaagt tggttaactt tttgtgtctc aatttcccca cttgtaaagt ggggctgggg 420
aaaaacccta cctcataggg aacattaagt gagttcatga aagccctcgg tgtgtggcat 480
gcgataacca ctacctaagc ataaggtatg tttacatgca ctggttgttt accacgacgc 540
accagcattc attcaagtta ttttcttcca gagtctgcct ggaatgtaga accggccagc 600
tctcctcctc aaacagttcc tgcaacctct ggttatctgt gtgggcagag gcgggagtgt 660
gggtgtgtgg ccctgccctg ggacagggat gacgagagag catgtgcttg tgtccagagc 720
tgtccccttc cctgctcagg ggccaccaaa tatgtccatg gtctctgctg cctggtcaac 780
ggccaccccg ggctgccgtc tcctggccag acctggagct cacaagctaa ttttagaaat 840
gactggtcga ctcagctgct gctatcacat tggcccagaa ggagggtggg gggaggacgg 900
gtcggggagg ggcagtaagg ggaagggatg tgtgtcggtg tgtgtggaga gggtgctcct 960
tcgctcctgc tttcccggct cggtggggtc ccttcgcggc                      1000